# EUROPEAN PATENT SPECIFICATION

(11) **EP 0 547 231 B1**
(45) Date of publication and mention of the grant of the patent: **28.08.1996**
(21) Application number: 92913882.4
(22) Date of filing: 24.06.1992
(51) Int. Cl.: C07C 65/24, A61K 31/21, C07C 69/92, C07C 69/94, C07C 229/36, C07C 271/22, C07C 317/46, C07C 323/62, A61K 31/35, C07D 309/38, C07D 493/10

(54) **PHOSPHOLIPASE A2 INHIBITOR**
PHOSPHOLIPASE A2 INHIBITOR
INHIBITEUR DE LA PHOSPHOLIPASE A2

(30) Priority: 03.07.1991 JP 162847/91
(43) Date of publication of application: 23.06.1993
(73) Proprietor: SHIONOGI & CO., LTD., Osaka 541 (JP)
(72) Inventor: OHTANI, Mitsuaki, Nara-shi Nara 630 (JP); MATSUTANI, Shigeru, Hashimoto-shi Wakayama 648 (JP); YOSHIDA, Tadashi, Toyono-gun Osaka 563-01 (JP); TANAKA, Kazushige Shionogi Seiyaku Terae-ryou, Amagasaki-shi Hyogo 660 (JP); FUJII, Yasuhiko, Kita-ku Kobe-shi Hyogo 651-15 (JP); SHIRAHASE, Kazuhiro, Neyagawa-shi Osaka 572 (JP)
(74) Representative: Baverstock, Michael George Douglas
(86) International application number: JP9200802
(87) International publication number: WO9301157

(56) References cited:
- EP-A- 0 395 418
- DE-A- 2 101 836
- DE-A- 2 205 932
- JP-B-43 029 941
- JOURNAL OF ANTIBIOTICS vol. 44, no. 12 , December 1991 , TOKYO JP pages 1467 - 1470 T YOSHIDA ET AL 'THIELOCINS A1alpha AND A1beta,NOVEL PHOSPHOLIPIDASE A2 INHIBITORS FROM ASCOMYCETES'
- CHEMICAL ABSTRACTS, vol. 117, no. 17, 26 October 1992, Columbus, Ohio, US; abstract no. 169572, page 670 ;column 2 ;
- CHEMICAL ABSTRACTS, vol. 117, no. 21, 23 November 1992, Columbus, Ohio, US; abstract no. 210731, page 683 ;column 1 ;
- The Merck Index (edit. No. 9), Merck & Co., Inc. (US), issued 1976, page 1147, Succinic Acid (8668), page 740, Maleic Acid (5531), page 552, Fumaric Acid (4137), page 682, Isophthalic Acid (5058), page 257, Chelidonic Acid (2008).
- CHEMICAL ABSTRACTS, Vol 86, No. 11, Abstract No. 72110j.
- CHEMICAL ABSTRACTS, Vol. 95, No. 11, Abstract No. 96666g.
- CHEMICAL ABSTRACTS, Vol. 72, No. 17, Abstract No. 89981c.

## Description

The present invention relates to a novel phospholipase A₂ inhibitor. In particular, the present invention relates to novel compounds exhibiting an inhibiting effect on phospholipase A₂, which compounds are analogs of physiologically active substances, thielocins which are produced by microorganisms such as Thielavia terricola RF-143 belonging to the Thielavia genus.

Phospholipase A₂ is an enzyme which exists in cells and secretory liquids, in particular, the venom of snakes, the pancreas of mammals, blood platelets of various animals, the arthritis exudate of higher animals, and elsewhere. The enzyme specifically hydrolyzes phospholipids. For example, the enzyme specifically hydrolyzes C-2 fatty acid esters of 1,2-diacylglycerol phospholipids to form lysoglycerophospholipids and fatty acids. Phospholipid A₂ exhibits toxicity on nerves, muscles and the heart, and anticoagulant action in association with the above enzymatic action, and it is generally said that the enzyme may induce, for example, convulsing, hypotonia, haemolysis, and edema. Furthermore, the enzyme can also be responsible for other clinical symptoms including inflammation. It should be noted that phospholipase A₂ is recognized to be one of the phlogogenic substances in humans.

If the enzymatic activity of phospholipase A₂, which is a phlogogenic substance, can be inhibited, various diseases caused by or associated with the enzymatic activity can probably be treated. Based on such an assumption, substances such as mepacrine and p-bromophenacyl bromide have already been developed, and the applicants have also claimed and described novel phospholipase A₂ inhibitors in Japanese Patent Publication (kokai) No. 286088/1990 and Japanese Patent Application No. 234955/1990. However, it is desirable to develop additional phospholipase A₂ inhibitors, because types of the phospholipase A₂ molecules are varied and the activity of one molecule is not the same as another due to the difference of the structure of the molecules.

The applicants have filed the Japanese patent applications which claim and describe for example, thielocin A₁α, A₁β, which is produced by Thielavia terricola RF-143 (Japanese Patent Application No. 109939/1989, and others). Now the applicants have chemically synthesized various new thielocin derivatives which are useful as medicines.

Specifically, the present invention relates to thielocin derivatives of the formula: 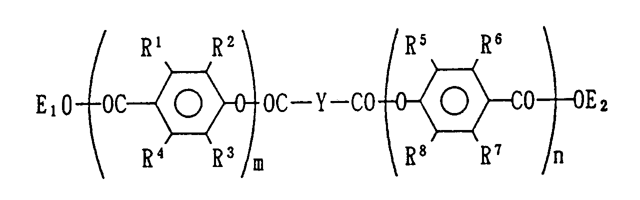 wherein R¹, R², R³, R⁴, R⁵, R⁶. R⁷, and R⁸ are independently hydrogen, lower alkyl, lower a;koxy, hydroxy, or halogen;
E₁ and E₂ are independently hydrogen, or an ester residue;
m and n are independently an integer of 0 to 4;
-Y- is a bivalent group which is selected from the following radicals:
-CH₂CH₂- , -CH=CH- , 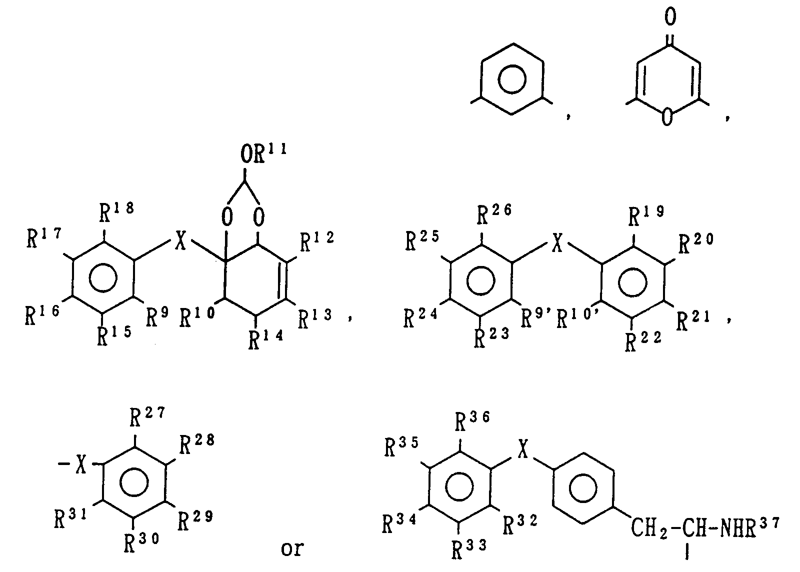 provided that m + n is not zero, when Y is -CH₂CH₂-, -CH=CH-, 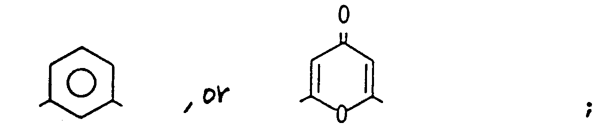 wherein X is a single bond, CH₂, O, S, SO, or SO₂;
R⁹ and R¹⁰ are independently a single bond, hydrogen, lower alkyl, lower alkoxy, hydroxy, or halogen, or R⁹ and R¹⁰ may be combined together to form methylene, ether, sulfide, sulfinyl, or sulfone;
R^{9'} and R^{10'} are independently a single bond, hydrogen, lower alkyl, lower alkoxy, hydroxy, or halogen, or R^{9'} and R^{10'} may be combined together to form methylene, ether, sulfide, sulfinyl, or sulfone;
R¹¹ is hydrogen or lower alkyl;
R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, and R³¹ are independently a single bond, hydrogen, lower alkyl, lower alkoxy, hydroxy, or halogen,
provided that one of R⁹, R¹⁵, R¹⁶, R¹⁷ and R¹⁸, one of R¹⁰, R¹², R¹³ and R¹⁴, one of R^{9'}, R²³, R²⁴, R²⁵ and R²⁶, one of R^{10'}, R¹⁹, R²⁰, R²¹ and R²², and one of R²⁷, R²⁸, R²⁹, R³⁰ and R³¹ are a single bond;
R³², R³³, R³⁴, R³⁵, and R³⁶ are independently hydrogen, lower alkyl, lower alkoxy, hydroxy, halogen, or Z, and R³⁷ is hydrogen or an amino-protecting group, wherein Z is a single bond or a bivalent group of the formula: 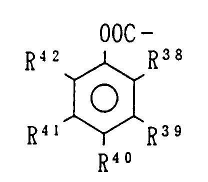 R³⁸, R³⁹, R⁴⁰, R⁴¹, and R⁴² are independently a single bond, hydrogen, lower alkyl, lower alkoxy, hydroxy, or halogen,
provided that one of R³², R³³, R³⁴, R³⁵ and R³⁶ is always Z, and when Z is not a single bond, one of R³⁸, R³⁹, R⁴⁰, R⁴¹ and R⁴² is a single bond, or when two or more of R³², R³³, R³⁴, R³⁵ and R³⁶ are Z, only one of Zs and R³⁸, R³⁹, R⁴⁰, R⁴¹ and R⁴² is a single bond;
or the salts thereof.

Several terms used in the present specification are defined below:

The term "lower alkyl" refers to C₁-C₆ alkyl such as methyl, ethyl, propyl, isopropyl, butyl, isobutyl, tert-butyl, pentyl and hexyl.

The term "lower alkoxy" refers to C₁-C₆ alkoxy such as methoxy, ethoxy, propoxy, isopropoxy, butoxy, isobutoxy, tert-butoxy, pentoxy and hexyloxy.

The term "halogen" refers to chlorine, bromine, iodine, or fluorine.

The term "ester residues" refers to an alkyl having 1 to 8 carbon atoms (e.g. methyl, methoxymethyl, ethyl, ethoxymethyl, iodoethyl, propyl, isopropyl, butyl, isobutyl, ethoxyethyl, methylthioethyl, methanesulfonylethyl, trichloroethyl and t-butyl), an alkenyl having 3 to 8 carbon atoms (e.g. propenyl, allyl, prenyl, hexenyl, phenylpropenyl and dimethylhexenyl), an aralkyl having 7 to 19 carbon atoms (e.g. benzyl, methylbenzyl, dimethylbenzyl, methoxybenzyl, ethoxybenzyl, nitrobenzyl, aminobenzyl, diphenylmethyl, phenylethyl, trityl, di-t-butylhydroxybenzyl, phthalidyl and phenacyl), an aryl having 6 to 12 carbon atoms (e.g. phenyl, toluyl, methoxyphenyl, diisopropylphenyl, xylyl, trichlorophenyl, pentachlorophenyl and indanyl), an ester formed with a N-hydroxyamino compound having 1 to 12 carbon atoms (e.g. esters formed with acetone oxime, acetophenone oxime, acetaldoxime, N-hydroxy succinimide and N-hydroxy phthalimide), a hydrocarbonated silyl having 3 to 12 carbon atoms (e.g. trimethyl silyl, dimethyl methoxy silyl and t-butyl dimethyl silyl), a hydrocarbonated stannyl having 3 to 12 carbon atoms (e.g. trimethyl stannyl), a mono-oxygenated alkyl having 2 - 15 carbon atoms e.g. a straight, branched, cyclic or partially cyclic alkanoyloxyalkyl such as acetoxymethyl, acetoxyethyl, propionyloxymethyl, pivaloyloxymethyl, pivaloyloxyethyl, cyclohexanacetoxyethyl, and cyclohexanecarbonyloxycyclohexylmethyl), an alkoxycarbonyloxyalkyl having 3 to 15 carbon atoms (e.g. ethoxycarbonyloxyethyl, isopropoxycarbonyloxyethyl, isopropoxycarbonyloxypropyl, t-butoxycarbonyloxyethyl, isopentyloxycarbonyloxypropyl, cyclohexyloxycarbonyloxyethyl, cyclohexylmethoxycarbonyloxyethyl, and bornyloxycarbonyloxyisopropyl), an alkoxyalkyl having 2 to 8 carbon atoms (e.g. methoxymethyl, and methoxyethyl), 2-oxacycloalkyl having 4 to 8 carbon atoms (e.g. tetrahydropyranyl and tetrahydrofuranyl esters), a substituted aralkyl having 8 to 12 carbon atoms (e.g. phenacyl and phthalidyl), an aryl having 6 to 12 carbon atoms (e.g. phenyl, xylyl, and indanyl), an alkenyl having 2 to 12 carbon atoms (e.g. allyl, and (2-oxo-1,3-disloyal) methyl). The protected group moiety may have any further substations.

An "amino-protecting group" includes those which are usually used in the art, and the preferred amino-protecting groups include acyl derivatives such as benzoyl, acetyl, formyl and trifluoroacetyl, urethane type derivatives such as benzyloxycarbonyl, t-butoxycarbonyl, isopropoxycarbonyl and methoxycarbonyl, or alkyl derivatives such as allyl, benzyl, trityl, and tetrahydropyranyl.

The compounds of the present invention can form salts with a metal such as an alkali metal (e.g. sodium or potassium), or an alkaline earth metal (e.g. calcium), which may form a salt with a carboxylic acid.

The compounds of the present invention can be prepared by coupling a dicarboxylic acid derivative of the formula:

R O C - Y - C O R'

wherein R and R' are independently hydroxy, halogen, or an ester residue, with an alcohol of the formula: 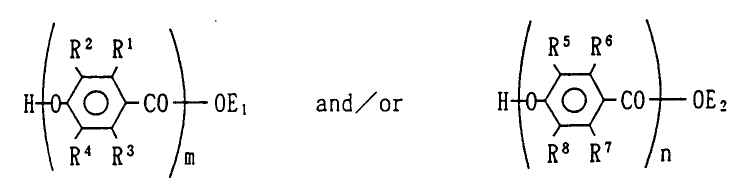 wherein R¹, R², R³, R⁴, R⁵, R⁶, R⁷,
R⁸, E₁ and E₂, m, and n are as defined above,
and if necessary, conducting a deesterification.

The present compounds can be formulated into oral or external preparations in association with various carriers. The dose of the compounds will differ depending on the intended treatment effect, the administration route, and age and body weight of particular patients, and therefore it is difficult to define the dose in general. On the whole, a daily dose may be about 0.1 mg to about 500 mg, preferably 0.5 mg to about 100 mg in the case of oral administration. On administration, the above dose may be divided into one to five portions.

Typical examples of the compounds of the present invention, which are shown in the above formula are illustrated below:

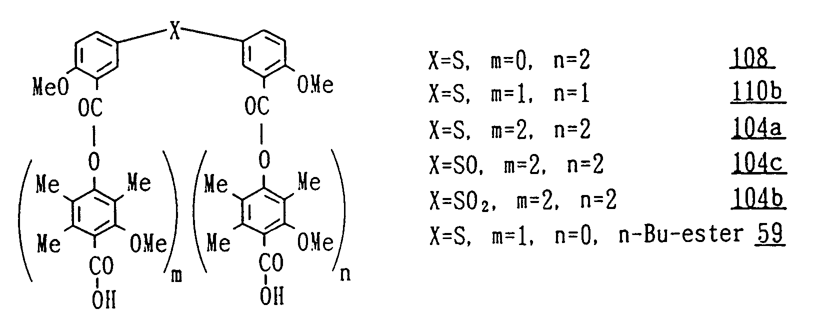

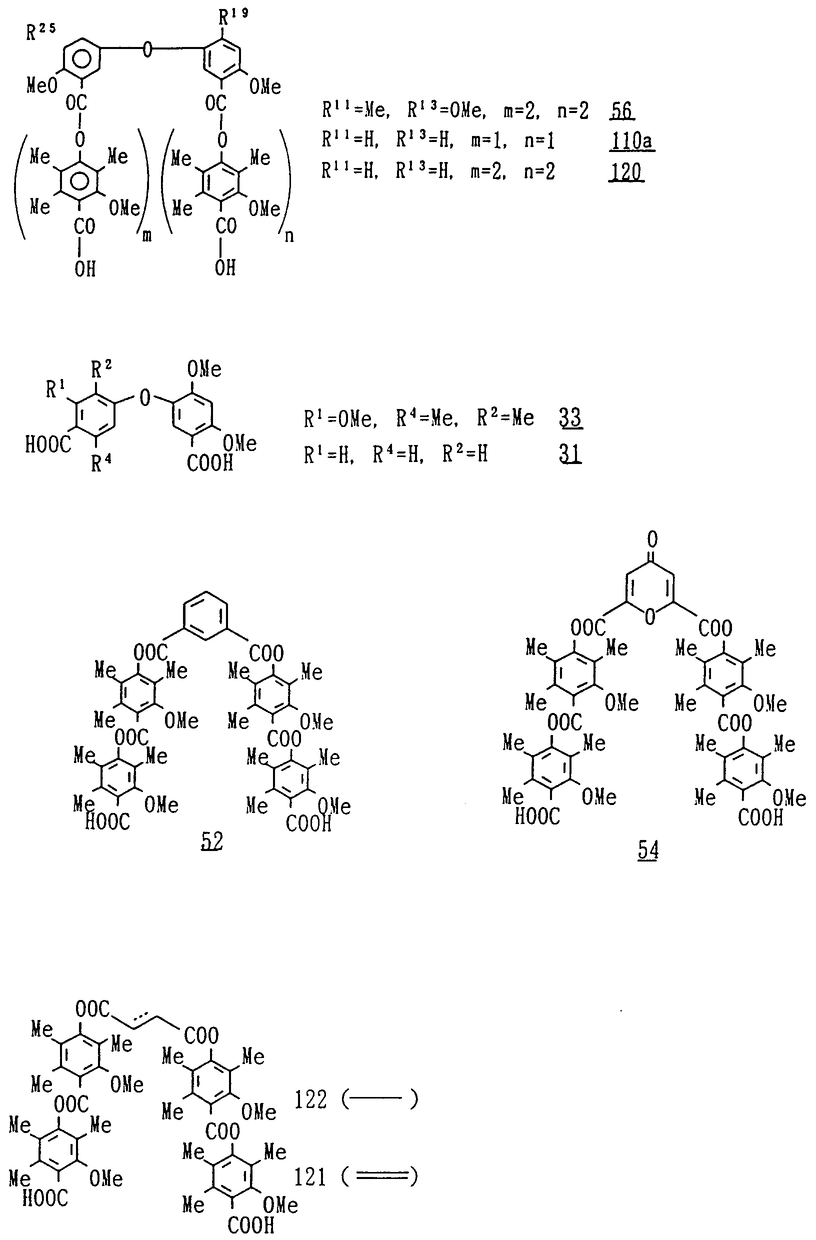

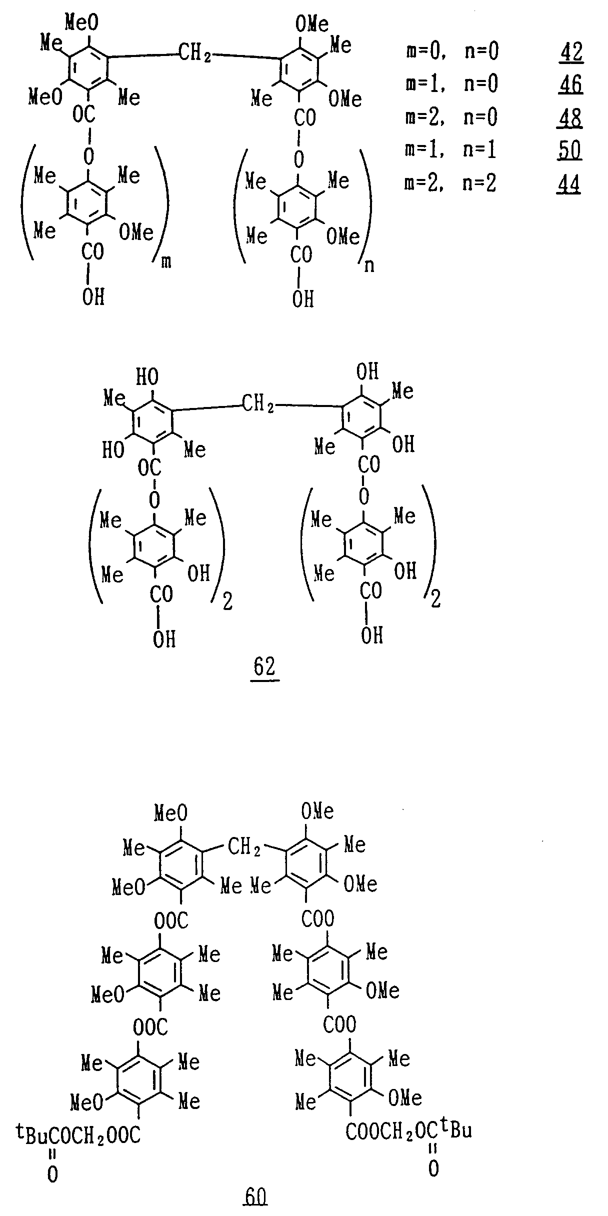

According to a further aspect of the present invention, there is provided another class of the following compounds which also exhibit phospholipase A₂ inhibitory effect:

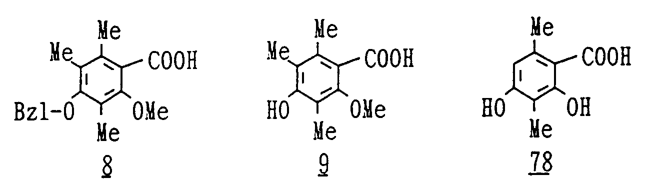

All of the compounds of the present invention can be prepared by various methods which are known in the art. The following Examples and Preparations are provided to further illustrate the process for preparing the compounds of the invention.

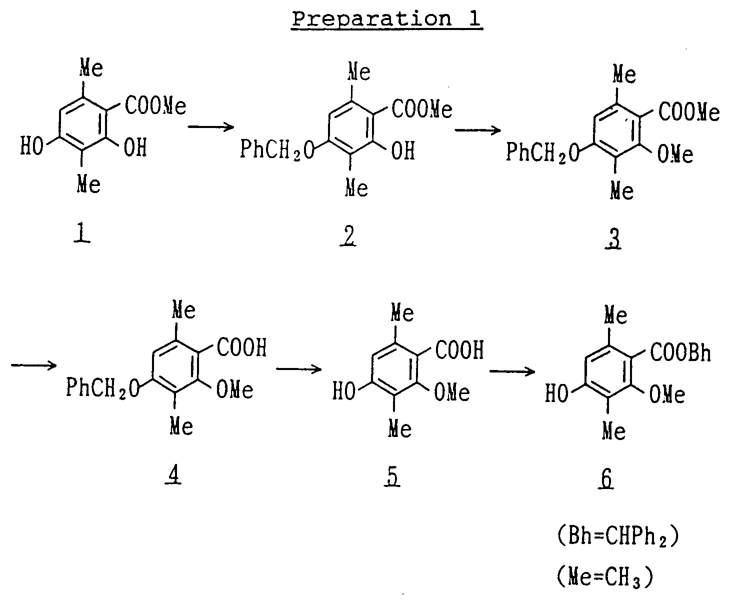

### [Step 1] (1→2)

A mixture containing the compound 1 (5.0 g, 25.5 mM), which is known in the literature, PhCH₂Br (3.2 ml, 25.5 mM x 1.06), K₂CO₃ (8.8 g, 25.5 mM x 2.5), and 200 ml of acetone was stirred at room temperature for 23 hours, and then the resultant solid was filtered off. The filtrate was distributed between ethyl acetate and 2 N hydrochloric acid, and the organic phase was washed with water, dried over Na₂SO₄, and then concentrated in vacuo to yield the crude compound 2. The compound was recrystallized from ether - n-hexane to provide 4.8 g of the benzylated compound 2 (66%) as colorless needle crystals. M.p: 77-78 °C. TLC (Rf: 0.6, Developer: n-hexane - ethyl acetate (4:1)).
- ¹HNMR (CDCl₃):: δ 2.15 (s,3H), 2.51 (s,3H), 3.92 (s,3H), 5.12 (s,2H), 6.34 (s,1H), 7.28-7.50 (m,5H), 11.84 (s,1H)
- IR (Nujol):: 1648, 1577, 803 cm⁻¹

| Elementary Analysis (for C₁₇H₁₈O₄) | | |
|---|---|---|
| Theory: | C,71.31; | H,6.34 (%) |
| Found : | C,70.99; | H,6.32 (%) |

### [Step 2](2→3)

A mixture containing the compound 2 (4.8 g, 16.8 mM), Me₂SO₄ (4.74 ml, 16.8 mM x 3), K₂CO₃ (11.58 g, 16.8 mM x 5), and 200 ml of acetone were heated under reflux for two hours, and, after cooling, the resultant solid was filtered off. The filtrate was distributed between ethyl acetate and 2 N hydrochloric acid, and the organic phase was washed with water, dried over Na₂SO₄, and then concentrated in vacuo to yield the crude product 3. The product was recrystallized from ether - n-hexane to provide 5.04 g of the compound 3 (100%) as colorless needle crystals. M.p: 56-57°C. TLC (Rf: 0.5, Developer: n-hexane - ethyl acetate (4:1)).
- ¹HNMR (CDCl₃):: δ 2.17 (s,3H), 2.29 (s,3H), 3.77 (s,3H), 3.90 (s,3H), 5.07 (s,2H), 6.54 (s,1H), 7.24-7.50 (m,5H)
- IR (Nujol);: 1725, 1605, 1580, 760, 703 cm⁻¹

| Elementary Analysis (for C₁₈H₂₀O₄) | | |
|---|---|---|
| Theory: | C,71.98; | H,6.71 (%) |
| Found : | C,71.97; | H,6.90 (%) |

### [Step 3] (3→4)

Compound 3 (5.04 g, 16.8 mM) was dissolved in 68.2 ml of DMSO, and KOH (5.64 g, 16.8 mM x 6) which had been dissolved in 13.6 ml of water at 0°C was added to the solution, and then the mixture was stirred overnight at 90^{o}C. After cooling, the mixture was poured into 2 N hydrochloric acid with ice, and the resultant mixture was extracted with ether. The organic phase was washed with water, and then dried, and concentrated in vacuo to yield the crude product 4. The product was recrystallized from ether - n-hexane to provide 4.44 g of the compound 4 (93%) as colorless flaky crystals. M.p: 124-125°C. TLC (Rf: 0.3, Developer: chloroform - methanol (9:1)).
- ¹HNMR (CDCl₃):: δ 2.20 (s,3H), 2.56 (s,3H), 3.85 (s,3H), 5.12 (s,2H), 6.65 (s,1H), 7.25-7.50 (m,5H)
- IR (Nujol):: 2200-3360, 1685, 1600, 1567, 1170, 1115 cm⁻¹

| Elementary Analysis (for C₁₇H₁₈O₄) | | |
|---|---|---|
| Theory: | C,71.31; | H,6.34 (%) |
| Found : | C,71.30; | H,6.44 (%) |

### [Step 4] (4→5)

A mixture containing the compound 4 (4.34 g, 15.2 mM), Pd-C (800 mg), 25 ml of ethyl acetate, and 6 ml of methanol was subjected to hydrogenation by passing hydrogen gas (340 ml, 15.2 mM) through the mixture.
After the catalyst was filtered off, the solution was concentrated in vacuo to yield 2.97 g of the colorless crystals 5 (100%). M.p: 153-155°C. TLC (Rf: 0.25, Developer: chloroform methanol (9:1)).
- ¹HNMR (CD₃0D):: δ 2.08 (s,3H), 2.24 (s,3H), 3.74 (s,3H), 6.44 (s,1H)
- IR (Nujol):: 2200-3440, 3200, 1715, 1610, 1588, 1263, 1155 cm⁻¹

| Elementary Analysis (for C₁₀H₁₂O₄) | | |
|---|---|---|
| Theory: | C,61.22; | H,6.17 (%) |
| Found : | C,61.02: | H,6.22 (%) |

### [Step 5] (5→6)

Compound 5 (2.97 g, 15.2 mM) was dissolved in 30 ml of ethyl acetate, and diphenyldiazomethane (5.89 g, 2 mM x 2) was added thereto at room temperature, and the mixture was left to stand overnight. An excess of diphenyldiazomethane was decomposed by adding 2 N hydrochloric acid thereto, and the resultant mixture was extracted with ethyl acetate. The organic phase was washed with water, dried, and then concentrated in vacuo. The residue was subjected to column chromatography (80 g of SiO₂, eluent: n-hexane - ethyl acetate (19:1) to (1:1)) to yield the benzhydryl ester 6. The product was recrystallized from ether - n-hexane to yield 3.76 g of 6 as colorless crystals (69%). M.p: 96-97°C. TLC (Rf: 0.25, Developer: n-hexane - ethyl acetate (4:1)).
- ¹HNMR (CDCl₃):: δ 2.12 (s,6H), 3.54 (s,3H), 4.97 (s,1H), 6.39 (s,1H), 7.15 (s,1H), 7.21-7.50 (m,10H)
- IR (Nujol):: 3410, 1686, 1610, 1590, 1160, 1095 cm⁻¹

| Elementary Analysis (for C₂₃H₂₂O₄) | | |
|---|---|---|
| Theory: | C,76.22; | H,6.12 (%) |
| Found : | C,76.31; | H,6.12 (%) |

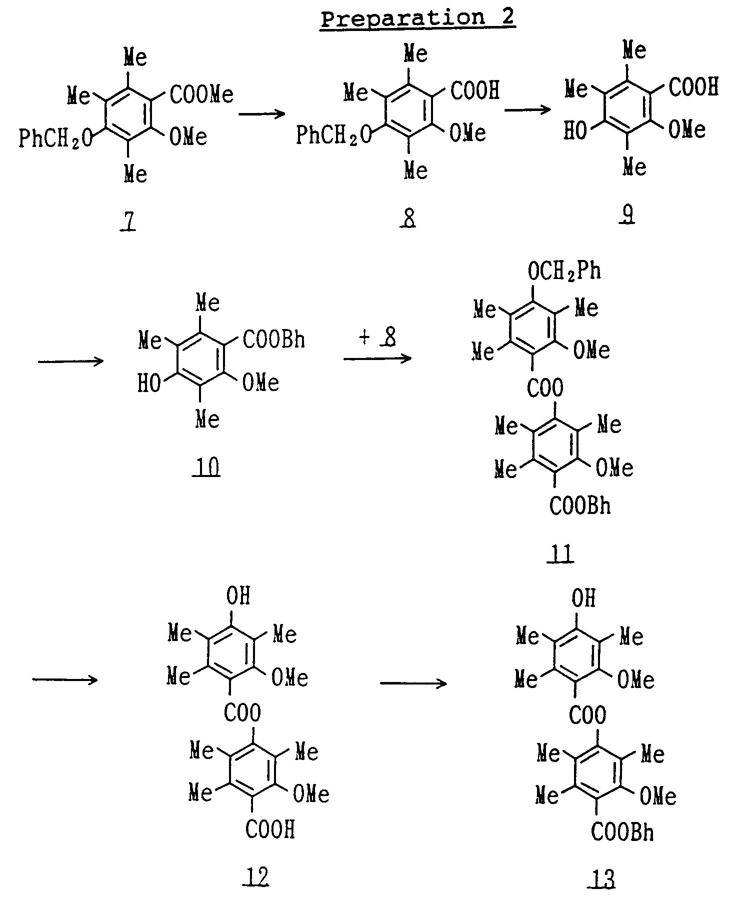

### [Step 1] (7→8)

Compound 7 (12.7 g, 40.4 mM), which is known in the literature, was hydrolyzed in a procedure similar to that of Step 3 in Preparation 1, to yield 11.7 g of the compound 8 as colorless crystals (97%). M.p. 122-130°C
- ¹HNMR (CDCl₃):: δ 2.22 (s,3H), 2.23 (s,3H), 2.33 (s,3H), 3.82 (s,3H), 4.78 (s,2H), 7.28-7.55 (m,5H)
- IR (KBr):: 3650-2250, 3430, 2950, 1687, 1222, 1105, 737, 693 cm⁻¹

| Elementary Analysis (for C₁₈H₂₀O₄) | | |
|---|---|---|
| Theory: | C,71.98; | H,6.71 (%) |
| Found : | C,72.17; | H,6.76 (%) |

### [Step 2] (8→9)

Compound 8 (5.0 g, 16.6 mM) was subjected to hydrogenation by a similar procedure to that of Step 4 in Preparation 1, to yield 3.8 g of the compound 9 as colorless crystals (97%). M.p.130-132°C. TLC (Rf: 0.4, Developer: chloroform - methanol (9:1)).
- ¹HNMR (CDCl₃):: δ 2.16 (s,3H), 2.19 (s,3H), 2.35 (s,3H), 3.81 (s,3H)
- IR (KBr):: 3660-2100, 3320, 1714, 1562, 1380, 1205 cm⁻¹

| Elementary Analysis (for C₁₁H₁₄O₄) | | |
|---|---|---|
| Theory: | C,62.85; | H,6.71 (%) |
| Found : | C,62.58; | H,6.65 (%) |

### [Step 3] (9→10)

Compound 9 (3.36 g, 16.0 mM) was benzhydrylated by a procedure similar to that of Step 5 in Preparation 1, to yield 5.76 g of the ester 10 (96%) as colorless pillar crystals. M.p.125-127 °C. TLC (Rf: 0.2, Developer: n-hexane - ethyl acetate (4:1)).
- ¹HNMR (CDCl₃):: δ 2.06 (s,3H), 2.11 (s,3H), 2.15 (s,3H), 3.52 (s,3H), 4.82 (s,1H), 7.17 (s,1H), 7.22-7.50 (m,10H)
- IR (Nujol):: 3360, 1695, 1585, 1290, 1205 cm⁻¹

| Elementary Analysis (for C₂₄H₂₄O₄) | | |
|---|---|---|
| Theory: | C,76.57; | H,6.43 (%) |
| Found : | C,76.49; | H,6.55 (%) |

### [Step 4] (8+10→11)

The compound 8 (4.19 g, 13.28 mM x 1.05) was dissolved in 20 ml of CH₂Cl₂, and oxalyl chloride (4.25 ml, 13.28 mM x 1.05 x 3.5) was added thereto at room temperature, and the resultant mixture was stirred at room temperature for 30 minutes, and then the mixture was gently warmed under reflux for 30 minutes. After concentration in vacuo, the residue was dissolved in THF, and the solution was again concentrated in vacuo. Alternatively, compound 10 (5.00 g, 13.28 mM) was dissolved in 20 ml of THF, and n-BuLi (1.6 M solution in hexane, 8.30 ml, 13.28 mM) was added gradually to the solution at -78 °C, and then the solution was stirred at the same temperature for 30 minutes. To the reaction mixture was added a solution of the acid chloride 8 obtained above in THF (30 ml) at -78 °C, and the mixture was stirred at the same temperature for 10 minutes. The mixture was allowed to warm slowly to room temperature, and left to stand overnight. The reaction mixture was distributed between ethyl acetate and 1 N hydrochloric acid, and the organic phase was washed with water, and then dried. After the mixture was concentrated in vacuo, the residue was subjected to column chromatography (200 g of SiO₂, eluent: toluene - ethyl acetate (0%-10%)) to yield 7.32 g of 11 as colorless crystals (84%). M.p: 183-185 °C. TLC (Rf: 0.7, Developer: benzene - ethyl acetate (9:1)).
- ¹HNMR (CDCl₃):: δ 2.09 (s,3H), 2.22 (s,3H), 2.25 (s,3H), 2.26 (s,3H), 2.28 (s,3H), 2.37 (s,3H), 3.58 (s,3H), 3.81 (s,3H), 4.80 (s,2H), 7.20 (s,1H), 7.28-7.55 (m,15H)
- IR (Nujol):: 1760, 1723, 1573, 1455, 1156, 740, 697 cm⁻¹

| Elementary Analysis (for C₄₂H₄₂O₇) | | |
|---|---|---|
| Theory: | C,76.57; | H,6.43 (%) |
| Found : | C,76.83; | H,6.55 (%) |

### [Step 5] (11→12)

Compound 11 (7.32 g, 11.1 mM) was subjected to hydrogenation by a procedure similar to that of Step 4 in Preparation 1, and the resultant crude compound 12 was recrystallized from toluene - n-hexane to yield 4.31 g of 12 as colorless crystals (96%). M.p. 207-209 °C. TLC (Rf: 0.2, Developer: chloroform - methanol (9:1)).
- ¹HNMR (DMSO):: δ 2.10-2.30 (m,18H), 3.69 (s,3H), 3.71 (s,3H), 8.70-8.93 (brs,1H)
- IR (KBr):: 3650-2260, 3500, 1750, 1576, 1460, 1410, 1170 cm⁻¹

| Elementary Analysis (for C₂₂H₂₆O₇) | | |
|---|---|---|
| Theory: | C,65.66; | H,6.51 (%) |
| Found : | C,65.50; | H,6.58 (%) |

### [Step 6] (12→13)

Compound 12 (4.31 g, 10.7 mM) was benzhydrylated by a procedure similar to that of Step 5 in Preparation 1, and the resultant compound was recrystallized from ether - n-hexane to yield 5.31 g of 13 (87%) as colorless pillar crystals. M.p. 195-197 °C. TLC (Rf: 0.4, Developer: toluene - ethyl acetate (9:1)).
- ¹HNMR (CDCl₃):: δ 2.08 (s,3H), 2.19-2.24 (m,12H), 2.37 (s,3H), 3.56 (s,3H), 3.80 (s,3H), 4.95 (s,1H), 7.19 (s,1H), 7.28-7.52 (m,10H)
- IR (Nujol):: 3390, 1737, 1706, 1285, 1156, 759, 700 cm⁻¹

| Elementary Analysis (for C₃₅H₃₆O₇) | | |
|---|---|---|
| Theory: | C,73.92; | H,6.38 (%) |
| Found : | C,73.75; | H,6.41 (%) |

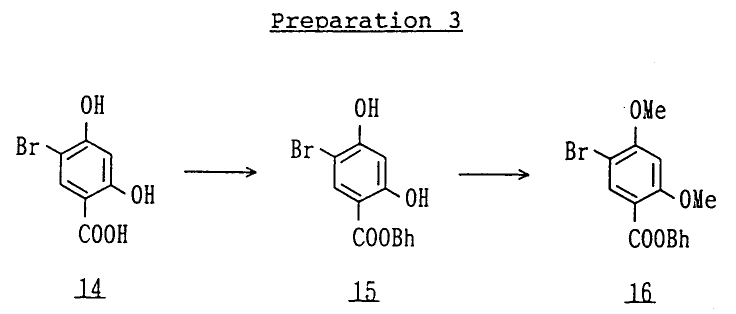

### 14→16

5-Bromo-2,4-dihydroxy benzoic acid, mono-hydrate 14 (10 g, 39.8 mM) was benzhydrylated by a procedure similar to that of Step 5 in Preparation 1, to yield 15, and the latter compound was dimethoxylated by the procedure of Step 2 in Preparation 1 to yield 16 as crude cystals. The resultant compound was recrystallized from ether - n-hexane to yield 13.6 g of 16 (80%) as colorless pillar crystals. M.p. 124-125 °C. TLC (Rf: 0.2, Developer: n-hexane - ethyl acetate (4:1)).
- ¹HNMR (CDCl₃):: δ 3.94 (s,3H), 3.95 (s,3H), 6.48 (s,1H), 7.07 (s,1H), 7.21-7.48 (m,10H), 8.16 (s,1H)
- IR (Nujol):: 1708, 1600, 1248, 1214, 1029 cm⁻¹

| Elementary Analysis (for C₂₂H₁₉O₄Br) | | | |
|---|---|---|---|
| Theory: | C,61.84; | H,4.48; | Br,18.70 (%) |
| Found : | C,61.85; | H,4.61; | Br,18.43 (%) |

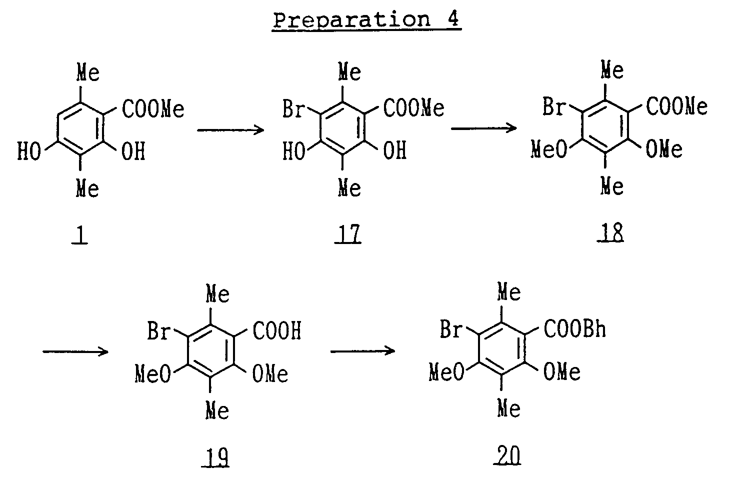

### [Step 1] (1→17)

Compound 1 (5.0 g, 25.5 mM), which is known in the literature , was suspended in 50 ml of 1,2-dichloroethane, and 10 ml of a solution of bromine (945 µl, 25.5 mM x 1.2) in 1,2-dichloroethane was added to the suspension, and the mixture was stirred overnight. The mixture was diluted with chloroform, and then the solution was washed with water, 5% aqueous sodium sulfite, and water successively in this order, and then dried. After concentration in vacuo the residue was subjected to column chromatography (90 g of SiO₂, eluent: n-hexane - ethyl acetate (9:1) to (2:1)) to yield 17. The resultant compound was recrystallized from ether - n-hexane to yield 5.64 g of 17 (80%) of colorless pillar crystals. M.p. 87-88 °C. TLC (Rf: 0.75, Developer: n-hexane - ethyl acetate (4:1)).
- ¹HNMR (CDCl₃):: δ 2.18 (s,3H), 2.64 (s,3H), 3.95 (s,3H), 6.15 (s,1H), 11.63 (s,1H)
- IR (Nujol):: 3440, 3420, 1655, 1607, 1265 cm⁻¹

| Elementary Analysis (for C₁₀H₁₁O₄Br) | | | |
|---|---|---|---|
| Theory: | C,43.66; | H,4.03; | Br,29.05 (%) |
| Found : | C,43.63; | H,4.08; | Br,29.13 (%) |

### [Step 2] (17→19)

Compound 17 (5.72 g, 20.8 mM) was dimethoxylated by the same procedure as Step 2 in Preparation 1 to yield the crude compound 18, which was directly hydrolyzed by the same procedure of Step 3 in Preparation 1 to yield the crude compound 19. The compound was recrystallized from petroleum ether to yield 4.69 g of the compound 19 (78%) as colorless needle crystals. M.p. 115-116 °C. TLC (Rf: 0.7, Developer: 1% acetic acid - ethyl acetate).
- ¹HNMR (CDCl₃):: δ 2.28 (s,3H), 2.47 (s,3H), 3.82 (s,3H), 3.83 (s,3H)
- IR (Nujol):: 3380-2000, 1690, 1582, 1556, 1303, 1105, 680 cm⁻¹

| Elementary Analysis (for C₁₁H₁₃O₄Br) | | | |
|---|---|---|---|
| Theory: | C,45.70; | H,4.53; | Br,27.64 (%) |
| Found : | C,45.81; | H,4.62; | Br,27.37 (%) |

### [Step 3] (19→20)

Compound 19 (1.30 g, 4.50 mM) was benzhydrylated by a procedure similar to that of Step 5 in Preparation 1, and the resultant compound was subjected to column chromatography (60 g of SiO₂, eluent: n-hexane - ethyl acetate (10:0) to (9:1)) to yield 20 as crystals, which were recrystallized from ethyl acetate - hexane to yield 1.87 g of 20 (91%) of colorless pillar crystals. M.p. 136-137 °C. TLC (Rf: 0.6, Developer: n-hexane - ethyl acetate (4:1)).
¹HNMR (CDCl₃): δ 2.19 (s,3H), 2.24 (s,3H), 3.54 (s,3H), 3.78 (s,3H), 7.12 (s,1H), 7.24-7.46 (m,10H)
IR (Nujol): 1725, 1266, 1160, 743, 699 cm⁻¹

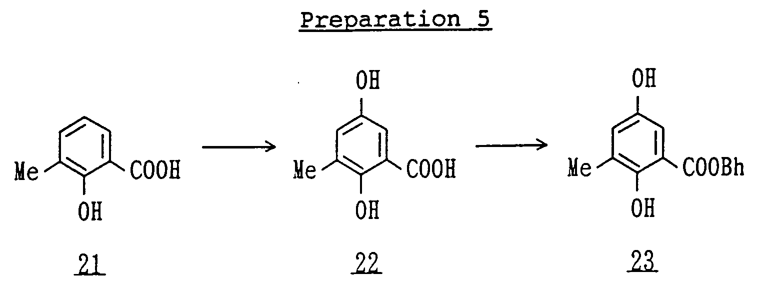

2-Hydroxy-3-methyl benzoic acid 21 (20 g, 131 mM) was dissolved in an aqueous sodium hydroxide (32 g of NaOH, 335 ml of water), and 260 ml of a solution of K₂S₂O₈ (40 g, 131 mM x 1.31) in water was added thereto at 0 °C. The reaction mixture was stirred at room temperature for four hours, and then left to stand for four days. The addition of dilute sulfuric acid (44 ml of sulfuric acid, 300 ml of water) resulted in the appearance of a brown precipitate. The precipitate was filtered off, and then the filtrate was heated under reflux for 9 hours. After cooling, the solution was partitioned by adding ethyl acetate and NaCl, and the organic phase was passed through a small amount of silica gel (40 g), and the resultant elution was concentrated to yield 22 as a brown crystalline powder. The powder was benzhydrylated by a procedure similar to that of Step 5 in Preparation 1, and the resultant compound was subjected to column chromatography (150 g of SiO₂, eluent: n-hexane - ethyl acetate (9:1)) to yield 15.2 mg of 23 (34.8%) as a pale yellow gummy material.
- ¹HNMR (CDCl₃):: δ 2.22 (s,3H), 4.55 (brs,1H), 6.90 (d,J=3.2Hz,1H), 7.09 (s,1H), 7.26-7.50 (m,11H), 10.50 (s,1H)

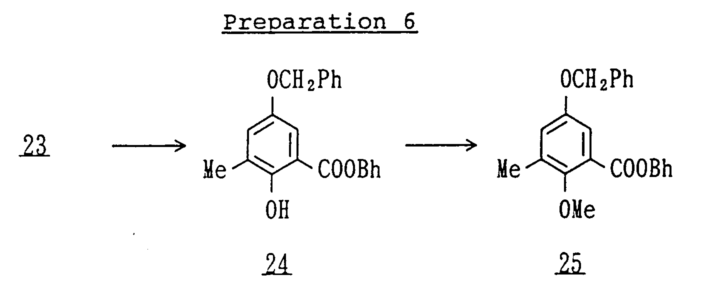

Compound 23 (7.6 g, 22.7 mM) was benzylated by a procedure similar to that of Step 1 in Preparation 1, to yield the crude compound 24. The compound was subjected to column chromatography (150 g of SiO₂, eluent: n-hexane - ethyl acetate (9:1)) to yield 8.1 g of 24 as a pale yellow oil (84.4%). Subsequently, the resultant compound was methoxylated by the procedure of Step 2 in Preparation 1 to yield the crude compound 25. The compound was subjected to column chromatography (150 g of SiO₂, eluent: n-hexane - ethyl acetate (9:1) to (4:1)) to yield 7.77 g of 25 as a pale yellow oil (92%). TLC (Rf: 0.4, Developer: n-hexane - ethyl acetate (4:1)).
- ¹HNMR (CDCl₃):: δ 2.29 (s,3H), 3.66 (s,3H), 5.03 (s,2H), 6.99 (d,J=3.0Hz,1H), 7.12 (s,1H), 7.20-7.50 (m,16H)

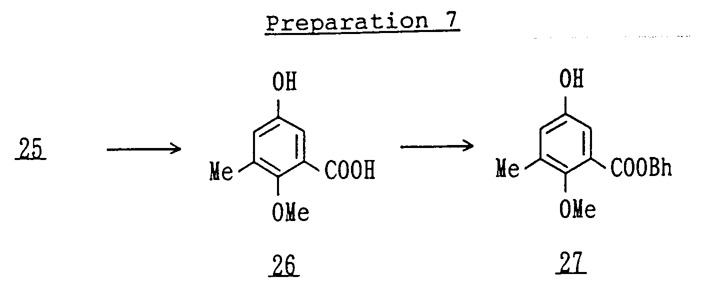

### [Step 1] (25→26)

Compound 25 (7.53 g, 17.2 mM) was subject to hydrogenation by a procedure similar to that of Step 4 in Preparation 1, to yield 26 as crude crystals. The compound was recrystallized from ether - petroleum ether to yield 2.83 g of 26 (90%) of colorless prism crystals. M.p. 142-144 °C. TLC (Rf: 0.5, Developer: ethyl acetate - acetic acid (1%)).
- ¹HNMR (DMSO):: δ 2.17 (s,3H), 3.64 (s,3H), 6.77 (d,J=3.0Hz,1H), 6.87 (d,J=3.0Hz,1H), 9.34 (brs,1H)
- IR (Nujol):: 3320, 1679, 1606 cm⁻¹

| Elementary Analysis (for C₉H₁₀O₄) | | |
|---|---|---|
| Theory: | C,59.34; | H,5.53 (%) |
| Found : | C,59.32; | H,5.56 (%) |

### [Step 2] (26→27)

Compound 26 (2.62 g, 14.4 mM) was benzhydrylated by a procedure similar to that of Step 5 in Preparation 1, to yield the crude compound 27. The compound was purified by column chromatography (150 g of SiO₂, eluent: n-hexane - ethyl acetate (19:1) to (2:1)), and the resultant compound was crystallized from ether - petroleum ether to yield 3.45 g of 27 (69%) as colorless prism crystals. M.p. 101-103 °C. TLC (Rf: 0.4, Developer: benzene - ethyl acetate (9:1)).
- ¹HNMR (CDCl₃):: δ 2.27 (s,3H), 3.66 (s,3H), 4.92 (s,1H), 6.85 (d,J=3Hz,1H), 7.12 (s,1H), 7.18 (d,J=3Hz,1H), 7.21-7.50 (m,10H)
- IR (Nujol):: 3430, 1689 cm⁻¹

| Elementary Analysis (for C₂₂H₂₀O₄) | | |
|---|---|---|
| Theory: | C,75.84; | H,5.79 (%) |
| Found : | C,75.89; | H,5.81 (%) |

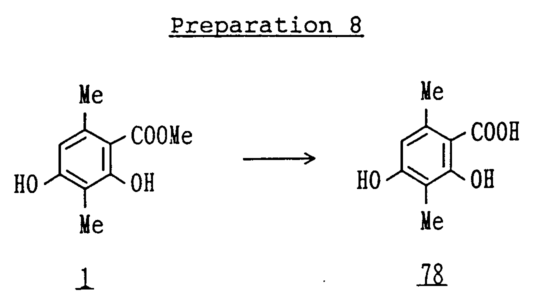

### Synthesis of 78

To compound 1 (1.00 g, 51.0 mM), which is known in the literature , was added ice-cooled conc. sulfuric acid (30 ml), and the mixture was left to stand overnight. The mixture was poured onto ice, and extracted with ether. The ether phase was extracted with aqueous saturated sodium bicarbonate, and the water phase was acidified with 2 N hydrochloric acid and extracted again with ether. The extract was washed with water, dried, and then concentrated in vacuo, to yield 7.63 g of 78 as colorless crystals (82%). M.p. 190-192 °C. TLC (Rf: 0.2, Developer: chloroform - methanol (9:1)).
- ¹HNMR (DMSO):: δ 1.93 (s,3H), 2.40 (s,3H), 3.36 (brs,1H), 6.26 (s,1H), 10.05 (s,1H), 12.90 (brs,1H)
- IR (KBr):: 3660-2080, 3410, 1639, 1458, 1262, 1175, 1091 cm⁻¹

| Elementary Analysis (for C₉H₁₀O₄) | | |
|---|---|---|
| Theory: | C,59.34; | H,5.53 (%) |
| Found : | C,59.25; | H,5.56 (%) |

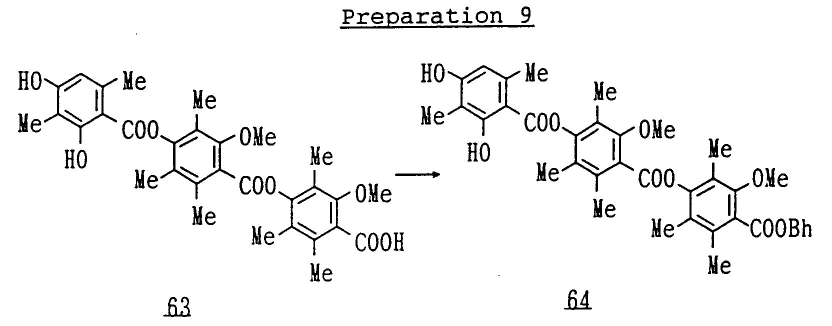

### Synthesis of 64

Compound 63 (1.00 g, 1.76 mM) was benzhydrylated by a procedure similar to that of Step 5 in Preparation 1, to yield the crude compound 64. The compound was crystallized from ethyl acetate - n-hexane to yield 765 mg of 64 as colorless crystals (59%). M.p. 198-200 °C. TLC (Rf: 0.2, Developer: n-hexane - ethyl acetate (4:1)).
¹HNMR (CDCl₃): δ 2.02-2.28 (m,18H), 2.40 (s,3H), 2.65 (s,3H), 3.57 (s,3H), 3.83 (s,3H), 5.44 (s,1H), 6.32 (s,1H), 7.20 (s,1H), 7.28-7.50 (m,10H), 11.86 (s,1H)

### Example 1

### 4-Carboxyphenyl (5'-carboxy-2',4'-dimethoxyphenyl) ether

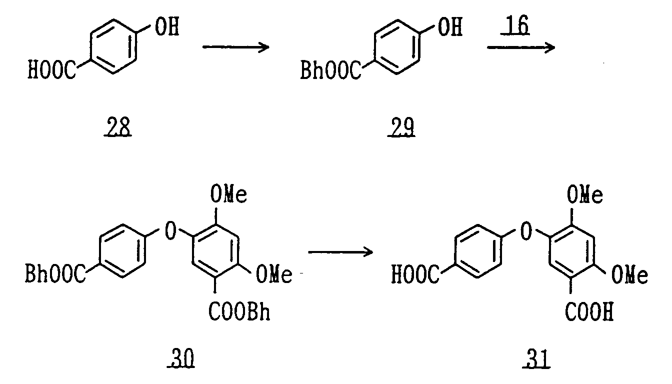

### [Step 1] (28→29)

4-Hydroxy benzoic acid 28 (2.76 g, 20 mM) was benzhydrylated in a procedure similar to that of Step 5 in Preparation 1, to yield the crude compound 29. The compound was recrystallized from methylene chloride - toluene to yield 4.47 g of 29 (74%).
M.p. 135-137 °C.
TLC (Rf: 0.2, Developer: n-hexane - ethyl acetate (4:1)).
- ¹HNMR (CDCl₃):: δ 5.63 (s,1H), 6.80-6.90 (m,A₂B₂ A-Part,2H), 7.08 (s,1H), 7.21-7.48 (m,10H), 7.98-8.12 (m,A₂B₂ B-Part,2H)

### [Step 2] (29+16→30)

Compound 29 (1.61 g, 5.29 mM) was dissolved in 15 ml of DMF, and to the solution was added NaH (212 mM, 5.29 mM) in a stream of argon at 0 °C. After the mixture was stirred at room temperature for 30 minutes, a complex of copper bromide dimethyl sulfide (3.26 g, 5.29 mM x 3) was added thereto, and the mixture was stirred for a while. Compound 16 (2.26 g, 5.29 mM) was added thereto, and then the mixture was stirred at 160 °C for about 20 hours. After cooling, the resultant mixture was distributed between ethyl acetate and 2 N hydrochloric acid, and the organic phase was washed with hydrochloric acid three times, water, and then dried, concentrated in vacuo, and the residue was subjected to column chromatography (70 g of SiO₂, eluent: n-hexane - ethyl acetate (9:1) to (1:1)), to yield 180 mg of 30 as an oil (5%).
TLC (Rf: 0.2, Developer: n-hexane - ethyl acetate (4:1)).
- ¹HNMR (CDCl₃):: δ 3.84 (s,3H), 3.97 (s,3H), 6.59 (s,1H), 6.85-6.94 (m,A₂B₂ A-part,2H), 7.06 (s,1H), 7.09 (s,1H), 7.18-7.48 (m,20H), 7.77 (s,1H), 8.03-8.12 (m,A₂B₂ B-part,2H)

### [Step 3] (30→31)

A mixture containing 30 (180 mg, 277 mM), trifluoroacetic acid (1 ml), and anisole (0.5 ml) was stirred at room temperature for 30 minutes, and after concentration in vacuo, the residue was crystallized by adding ether thereto, to yield 62 mg of 31 (70%).
M.p. 237-239 °C
TLC (Rf: 0.4, Developer: ethyl acetate - acetic acid (1%)).
- ¹HNMR (DMSO):: δ 3.86 (s,3H), 3.99 (s,3H), 6.80-6.90 (m,A₂B₂ A-part,2H), 6.85 (s,1H), 7.64 (s,1H), 7.90-8.00 (m,A₂B₂ B-part,2H)
- IR (KBr):: 3680-2320, 3300, 1725, 1675, 1618, 1230, 1023 cm⁻¹

| Elementary Analysis (for C₁₆H₁₄O₇) | | |
|---|---|---|
| Theory: | C,60.38; | H,4.43 (%) |
| Found : | C,60.12; | H,4.70 (%) |

### Example 2

### 4-Carboxy-3-methoxy-2,5-dimethylphenyl (5'-carboxy-2',4'-dimethoxyphenyl) ether

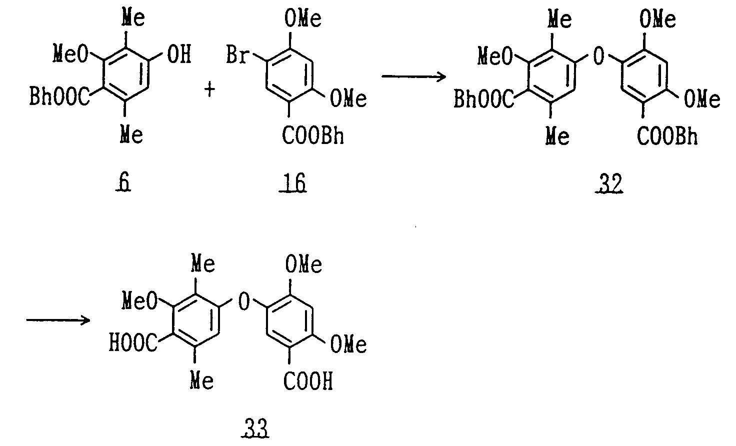

### [Step 1] (6+16→32)

Compound 6 (2.0 g, 5.52 mM), and 16 (2.36 g, 5.52 mM) were treated in a procedure similar to that of Step 2 in Example 1, to yield the crude compound 32. The compound was subjected to column chromatography (90 g of SiO₂, eluent: n-hexane - ethyl acetate (9:1) to (1:1)), to yield 670 mg of 32 as an oil (17%).
TLC (Rf: 0.2, Developer: n-hexane - ethyl acetate (4:1)).
- ¹HNMR (CDCl₃):: δ 2.04 (s,3H), 2.24 (s,3H), 3.58 (s,3H), 3.86 (s,3H), 3.96 (s,3H), 6.16 (s,1H), 6.58 (s,1H), 7.05 (s,1H), 7.16 (s,1H), 7.18-7.58 (m,20H), 7.63 (s,1H)

### [Step 2] (32→33)

Compound 32 (670 mg, 945 µM) was treated in a similar procedure to that of Step 3 in Expample 1, to yield 288 mg of 33 as colorless crystals (81%).
M.p. 199-200 °C.
TLC (Rf: 0.5, Developer: ethyl acetate - acetic acid (1%)).
- ¹HNMR (DMSO):: δ 2.10 (s,3H), 2.14 (s,3H), 3.73 (s,3H), 3.87 (s,3H), 3.89 (s,3H), 6.17 (s,1H), 6.86 (s,1H), 7.32 (s,1H)
- IR (KBr):: 3680-2730, 3430, 3260, 1731, 1688, 1611, 1512, 1440, 1423 cm⁻¹

| Elementary Analysis (for C₁₉H₂₀O₈·0.75H₂O) | | |
|---|---|---|
| Theory: | C,58.53; | H,5.56 |
| Found : | C,58.51; | H,5.25 |

### Example 3

### 3-Carboxy-4-methoxy-5-methylphenyl-(5,-carboxy-2',4'-dimethoxyphenyl) ether

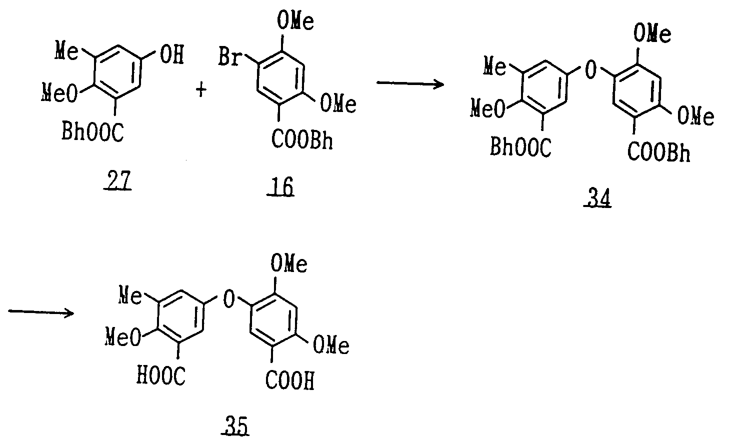

### [Step 1] (27+16→34)

Compound 27 (1.0 g, 2.87 mM), and 16 (1.23 g, 2.87 mM) were treated in a procedure similar to that of Step 2 in Expample 1, to yield the crude compound 34. The compound was subjected to column chromatography (80 g of SiO₂, eluent: n-hexane - ethyl acetate (4:1) to (2:1)), to yield 400 mg of 34 as an oil (20%).
TLC (Rf: 0.1, Developer: n-hexane - ethyl acetate (4:1)).
- ¹HNMR (CDCl₃):: δ 2.26 (s,3H), 3.67 (s,3H), 3.86 (s,3H), 3.96 (s,3H), 6.58 (s,1H), 6.87 (d,J=4Hz,1H), 7.05 (s,1H), 7.07 (s,1H), 7.10-7.54 (m,11H), 7.70 (s,1H)

### [Step 2] (34→35)

Compound 34 (320 mg, 461 µM) was treated in a procedure similar to that of Step 3 in Expample 1, to yield 112 mg of 35 as pale yellow crystals (67%). TLC (Rf: 0.3, Developer: ethyl acetate - acetic acid (1%)).
- ¹HNMR (DMSO):: δ 2.22 (s,3H), 3.69 (s,3H), 3.86 (s,3H), 3.90 (s,3H), 6.82 (d,J=3.0Hz,1H), 6.87 (s,1H), 6.95 (d,J=3.0Hz,1H), 7.39 (s,1H)
- IR (KBr):: 3680-2320, 3440, 2960, 1695, 1616, 1215, 1120 cm⁻¹

| Elementary Analysis (for C₁₈H₁₈O₈·0.3H₂O) | | |
|---|---|---|
| Theory: | C,58.79; | H,5.10 (%) |
| Found : | C,58.86; | H,5.09 (%) |

### Example 4

### Bis(3-carboxy-4,6-dimethoxy-2,5-dimethylphenyl) methane

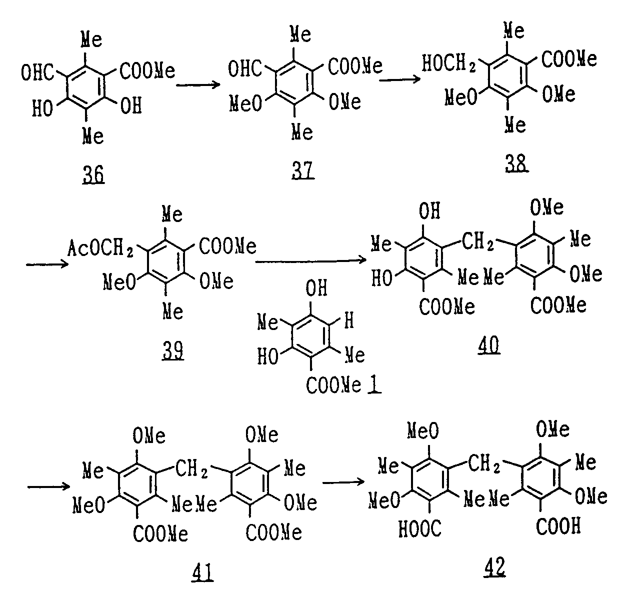

### [Step 1] Synthesis of acetate 39

The aldehyde 36 (3.70 g, 16.5 mM), which is described in the literature , was methoxylated by a similar procedure to that of Step 2 in Preparation 1, to yield 6.8 g of 37 as a red oil. The compound was dissolved in 30 ml of isopropyl alcohol, and to the solution was added NaBH₄ (0.94 g, 16.5 mM x 1.5), and the reaction mixture was stirred, and then it was distributed between ethyl acetate and water. The organic phase was washed with 2 N hydrochloric acid, followed by water, dried, and then concentrated in vacuo. The resultant residue was subjected to column chromatography (70 g of SiO₂, eluent: n-hexane - ethyl acetate (4:1) to (1:1)), to yield 3.18 g of the alcohol 38 (76% based on 1) as a colorless oil. TLC (Rf: 0.2, Developer: n-hexane - ethyl acetate (4:1)).

Thereafter, the oil was dissolved in 20 ml of CH₂Cl₂, and to the solution was added 6 ml of acetic anhydride, 10 ml of pyridine, and 50 mg of dimethylaminopyridine, and then the mixture was stirred at room temperature for two hours. The reaction mixture was distributed between ethyl acetate and 2 N hydrochloric acid, and the organic phase was washed with water, and dried, then concentrated in vacuo to yield the crude compound 39. The compound was recrystallized from ether - n-hexane to yield 3.35 g of the acetate 39 (91%) as colorless pillar-crystals. M.p. 69-70 °C. TLC (Rf: 0.4, Developer: n-hexane - ethyl acetate (4:1)).
- ¹HNMR (CDCl₃):: δ 2.07 (s,3H), 2.22 (s,3H), 2.24 (s,3H), 3.74 (s,3H), 3.78 (s,3H), 3.93 (s,3H), 5.18 (s,2H)

### [Step 2] Synthesis of 40

Compound 1 (1.45 g, 6.74 mM x 1.1), which is known in the literature and the acetate 39 (2.00 g, 6.74 mM) were dissolved in 25 ml of toluene, and to the solution was added BF₃·OEt₂ (0.83 ml, 6.74 mM), and the mixture was stirred at room temperature for 15 minutes. To the reaction mixture was added ethyl acetate, then the mixture was washed with water, dried, and then concentrated in vacuo. The residue was subjected to column chromatography (SiO₂; Merck, Lobar B, eluent: n-hexane - ethyl acetate), to yield 2.91 g of 40 (100%) as colorless prism crystals. M.p. 169-170 °C. TLC (Rf: 0.3, Developer: n-hexane - ethyl acetate (4:1)).
- ¹HNMR (CDCl₃):: δ 2.07 (s,6H), 2.25 (s,3H), 2.55 (s,3H), 3.75 (s,6H), 3.88 (s,3H), 3.92 (s,3H), 3.95 (s,2H), 7.20 (s,1H), 11.38 (s,1H).
- IR (Nujol):: 3285, 1743, 1644 cm⁻¹

| Elementary Analysis (for C₂₃H₂₈O₈) | | |
|---|---|---|
| Theory: | C,63.87; | H,6.54 (%) |
| Found : | C,63.65; | H,6.57 (%) |

### [Step 3] Synthesis of 41

Compound 40 (2.91 g, 7.4 mM) was methoxylated in a procedure similar to that of Step 2 in Preparation 1, and the crude residue was subjected to column chromatography (60 g of SiO₂, eluent: toluene - ethyl acetate (9:1) to (4:1)), to yield 2.97 g of 41 (96%) as colorless pillar crystals. M.p. 271-220 °C. TLC (Rf: 0.5, Developer: benzene - ethyl acetate (9:1)).
- ¹HNMR (CDCl₃):: δ 2.03 (s,6H), 2.20 (s,6H), 3.48 (s,6H), 3.74 (s,6H), 3.88 (s,6H), 4.03 (s,2H)
- IR (Nujol):: 1730, 1588, 1572, 1104 cm⁻¹

| Elementary Analysis (for C₂₅H₃₂O₈) | | |
|---|---|---|
| Theory: | C,65.19; | H,7.02 (%) |
| Found : | C,65.14; | H,7.04 (%) |

### [Step 4] (41→42)

Compound 41 (600 mg, 130 mM) was hydrolyzed in a procedure similar to that of Step 3 in Preparation 1, to yield 559 mg of 42 as colorless crystals (99%). M.p. 265-267 °C. TLC (Rf: 0.7, Developer: ethyl acetate - acetic acid - water (18:1:1)).
- ¹HNMR (DMSO):: δ 1.96 (s,6H), 2.12 (s,6H), 3.48 (s,6H), 3.66 (s,6H), 3.98 (s,2H)
- IR (KBr):: 3670-2400, 3430, 2940, 1710, 1217, 1105 cm⁻¹

| Elementary Analysis (for C₂₃H₂₈O₈·0.4H₂O) | | |
|---|---|---|
| Theory: | C,62.83; | H,6.60 (%) |
| Found : | C,62.88; | H,6.63 (%) |

### Example 5

### Bis[5-[4'-(4''-carboxy-3''-methoxy-2'',5'',6''-trimethylphenoxycarbonyl)-3'-methoxy-2',5',6',-trimethylphenoxycarbonyl]-2,4-dimethoxy-3,6-dimethylphenyl] methane

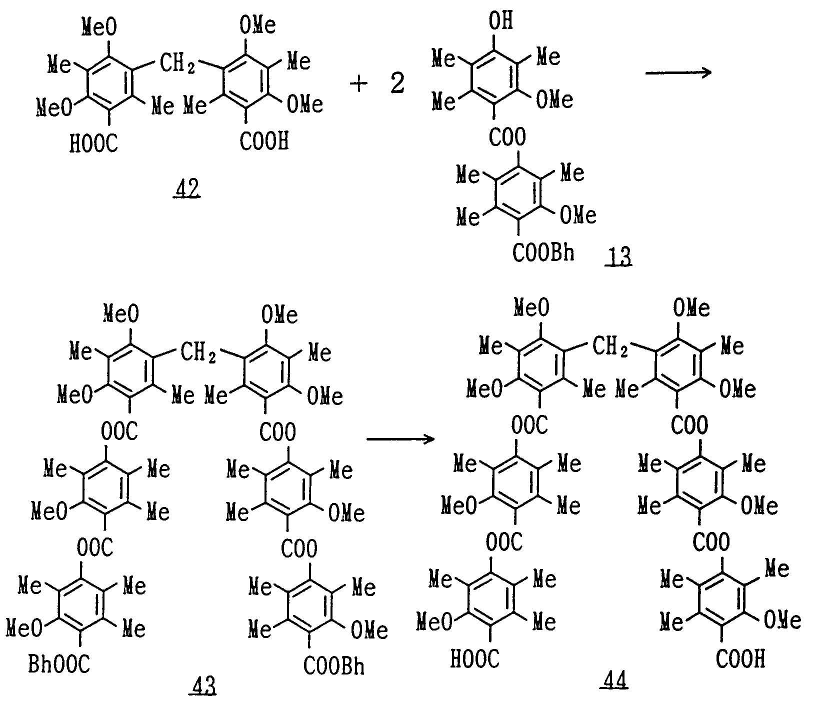

### [Step 1] Synthesis of 43

Compound 42 (100 mg, 231 µM) and 13 (263 mg, 231 µM x 2) were esterified in a procedure similar to that of Step 4 in Preparation 2, to yield the crude compound 43. The compound was subjected to column chromatography (15 g of SiO₂, eluent: n-hexane - ethyl acetate (4:1) to (2:1)), to yield 219 mg of 43 as colorless crystals (62%). M.p. 245-247 °C. TLC (Rf: 0.7, Developer: benzene - ethyl acetate (9:1)).
- ¹HNMR (CDCl₃):: δ 2.08 (s,6H), 2.20 (s,6H), 2.25 (s,12H), 2.30 (s,18H), 2.39 (s,6H), 3.57 (s,6H), 3.60 (s,6H), 3.83 (s,12H), 4.17 (s,2H), 7.19 (s,2H), 7.22-7.50 (m,20H)
- IR (Nujol):: 1745, 1150, 1097, 1074 cm⁻¹

| Elementary Analysis (for C₉₃H₉₆O₂₀) | | |
|---|---|---|
| Theory: | C,72.83; | H,6.31 (%) |
| Found : | C,72.74; | H,6.33 (%) |

### [Step 2] Synthesis of 44

Compound 43 (186 mg, 121 µM) was deprotected in a procedure similar to that of Step 3 in Example 1, to yield 140 mg of 44 as a colorless powder (96%). TLC (Rf: 0.4, Developer: ethyl acetate - acetic acid (1%)).
- ¹HNMR (DMSO):: δ 2.12-2.40 (m,48H), 3.54 (s,6H), 3.73 (s,6H), 3.77 (s,12H), 4.14 (s,2H)
- IR (KBr):: 3680-2400, 3450, 2940, 1743, 1697, 1140 cm⁻¹

| Elementary Analysis (for C₆₇H₇₆O₂₀) | | |
|---|---|---|
| Theory: | C,66.99; | H,6.38 (%) |
| Found : | C,66.73; | H,6.31 (%) |

### Example 6-11

The following compounds were synthesized as shown above.

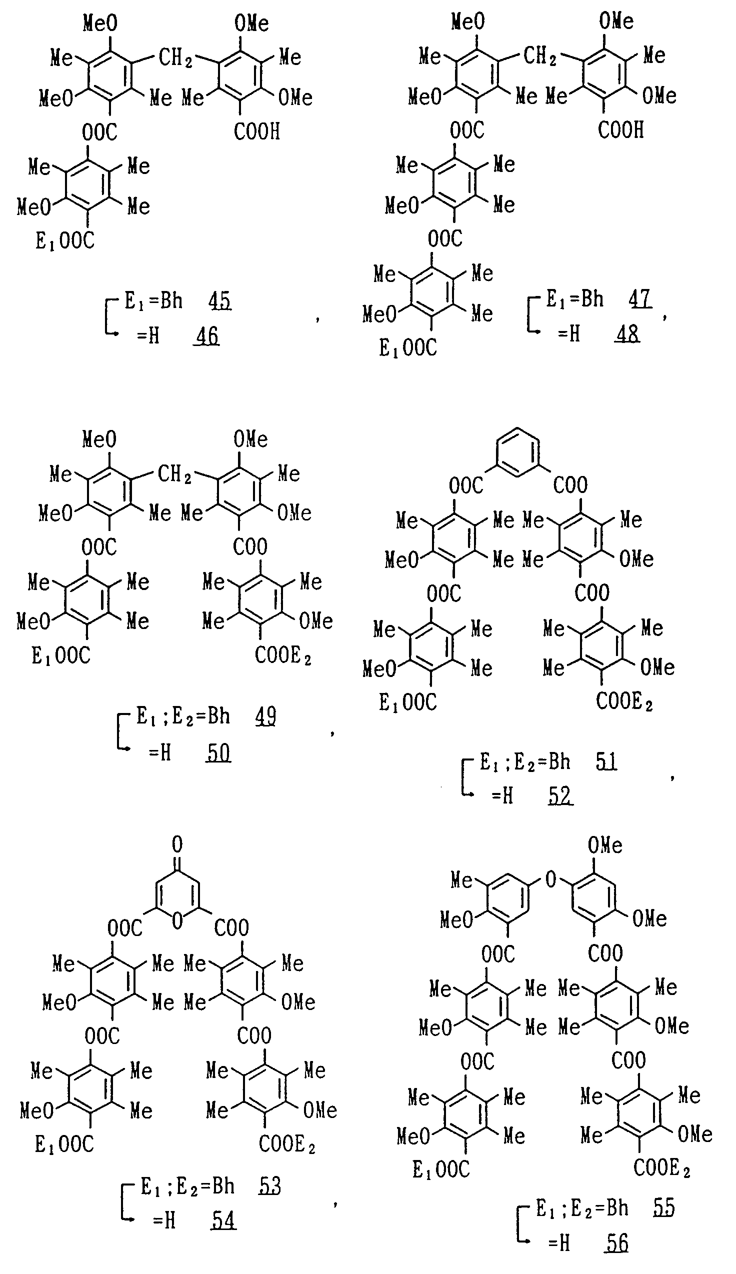

| Example | Compound | Starting Material | | Ratio (COOH:OH) | yield |
|---|---|---|---|---|---|
| | | COOH | OH | | |
| 6 | 46 | 42 | 10 | 1:1 | 17% |
| 7 | 48 | 42 | 13 | 1:1 | 20% |
| 8 | 50 | 42 | 10 | 1:2 | 70% |
| 9 | 52 | isophthalic acid | 13 | 1:2 | 60% |
| 10 | 54 | chelidonic acid | 13 | 1:2 | 27% |
| 11 | 56 | 35 | 13 | 1:2 | 42% |

### Physicochemical data

Compound 46; Colorless crystals. M.p. 141-143 °C. TLC (Rf: 0.4, Developer: ethyl acetate - acetic acid (1%)).
- ¹HNMR (CD₃OD):: δ 1.99 (s,3H), 2.10 (s,3H), 2.16-2.28 (m,15H), 3.54 (s,3H), 3.62 (s,3H), 3.77 (s,3H), 3.78 (s,3H), 3.80 (s,3H), 4.16 (s,2H)
- IR (KBr):: 3420, 2930, 1738, 1569, 1150, 1095 cm⁻¹

Compound 48; Colorless powder. TLC (Rf: 0.4, Developer: ethyl acetate - acetic acid (1%)).
- ¹HNMR (CD₃OD):: δ 2.00 (s,3H), 2.11 (s,3H), 2.18-2.34 (m,21H), 2.41 (s,3H), 3.57 (s,3H), 3.63 (s,3H), 3.77 (s,3H), 3.82 (s,3H), 3.83 (s,3H), 4.18 (s,2H)
- IR (KBr):: 3440, 2950, 1745, 1703, 1570, 1460, 1145, 1095, 1075 cm⁻¹

Compound 50; Colorless crystals. M.p. 267-269 °C. TLC (Rf: 0.4, Developer: ethyl acetate - acetic acid (1%)).
- ¹HNMR (DMSO):: δ 2.04-2.28 (m,30H), 3.52 (s,6H), 3.70 (s,6H), 3.74 (s,6H), 4.11 (s,2H)
- IR (KBr):: 3440, 2940, 1740, 1700, 1460, 1155 cm⁻¹

| Elementary Analysis (for C₄₅H₅₂O₁₄) | | |
|---|---|---|
| Theory: | C,66.16; | H,6.42 (%) |
| Found : | C,65.97; | H,6.48 (%) |

Compound 52; Colorless crystals. M.p. 267-269 °C. TLC (Rf: 0.4, Developer: ethyl acetate - acetic acid (1%)).
- ¹HNMR (DMSO):: δ 2.08-2.26 (m,30H), 2.37 (s,6H), 3.73 (s,6H), 3.79 (s,6H), 7.98 (t,J=9Hz,1H), 8.66 (dd,J=9Hz,J=1Hz,2H), 8.93 (m,1H)
- IR (KBr):: 3430, 2940, 1745, 1700, 1222, 1160 cm⁻¹

| Elementary Analysis (for C₅₂H₅₄O₁₆·H₂O) | | |
|---|---|---|
| Theory: | C,65.54; | H,5.92 (%) |
| Found : | C,65.44; | H,5.84 (%) |

Compound 54; Colorless plate crystals. M.p. 294-296 °C. TLC (Rf: 0.3, Developer: ethyl acetate - acetic acid (1%)).
- ¹HNMR (CDCl₃):: δ 2.16 (s,6H), 2.21 (s,6H), 2.26 (s,6H), 2.29 (s,6H), 2.36 (s,6H), 2.42 (s,6H), 3.84 (s,6H), 3.87 (s,6H), 7.52 (s,2H)

| Elementary Analysis (for C₅₁H₅₂O₁₈) | | |
|---|---|---|
| Theory: | C,64.27; | H,5.51 (%) |
| Found : | C,63.88; | H,5.57 (%) |

Compound 56; Colorless powder (hygroscopic). TLC (Rf: 0.3, Developer: ethyl acetate - acetic acid (1%)).
- ¹HNMR (DMSO):: δ 1.90 (s,3H), 1.98-2.90 (m,27H), 2.28-2.37 (m,9H), 3.68-3.81 (m,15H), 3.96 (s,3H), 4.00 (s,3H), 7.03 (s,1H), 7.18 (d,J=2.8Hz,1H), 7.28 (d,J=2.8Hz,1H), 7.80 (s,1H)
- IR (KBr):: 3430, 2930, 1745, 1575, 1462, 1150 cm⁻¹

| Elementary Analysis (for C₆₂H₆₆O₂₀·3.5H₂O) | | |
|---|---|---|
| Theory: | C,62.36; | H,6.16 (%) |
| Found : | C,62.31; | H,5.92 (%) |

### Example 12

### n-Butyl 3-[3'-(4''-carboxy-3''-methoxy-2'',5'',6''-trimethylphenoxycarbonyl)-4'-methoxyphenyl]-thio-6-methoxybenzoate

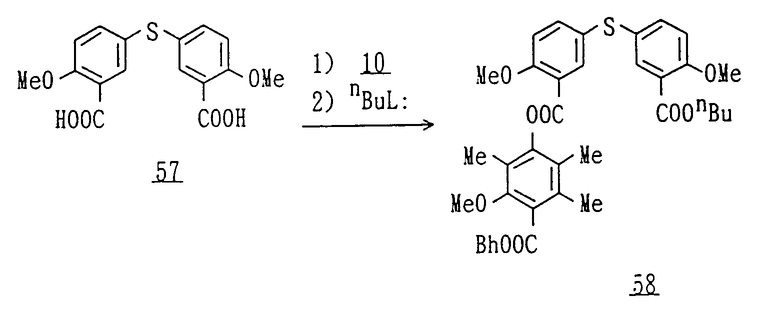

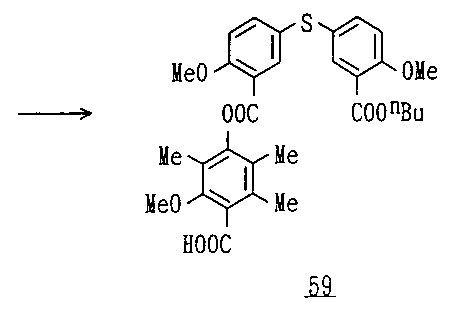

### Synthesis of 59

Compound 57 (50 mg, 150 µM) and 10 (56 mg, 150 µM) were treated in a procedure similar to that of Step 4 in Preparation 2, to yield the half-ester. The compound was reacted with n-butyl lithium (1.6 N solution in hexane, 93 µl, 150 µM), and the reaction mixture was worked up in a conventional manner to yield the crude compound 58. The compound was subjected to column chromatography (3 g of SiO₂, eluent: toluene - ethyl acetate (1:0) to (4:1)), to yield 58 as a colorless oil. The oil was deprotected in a procedure similar to that of Step 3 in Example 1 to yield 14 mg of 59 (16%) as a colorless oil.

TLC (Rf: 0.5, Developer: ethyl acetate - acetic acid (1%)).
- ¹HNMR (CDCl₃):: δ 0.95 (t,J=7.2Hz,3H), 1.30-1.53 (m,2H), 1.59-1.80 (m,2H), 2.08 (s,3H), 2.09 (s,3H), 2.30 (s,3H), 3.80 (s,3H), 3.89 (s,3H), 3.92 (s,3H), 4.27 (t,J=7Hz,2H), 6.90-7.05 (m,2H), 7.40-7.54 (m,2H), 7.82 (d,J=1.2Hz,1H), 8.01 (J=1.2Hz,1H)

### Example 13

### Bis[5-[4'-(4''-pivaloyloxymethyloxycarbonyl-3''-methoxy-2'',5'',6''-trimethylphenoxycarbonyl)-3'-methoxy-2',5',6'-trimethylphenoxycarbonyl]-2,4-dimethoxy-3,6-dimethylphenyl] methane

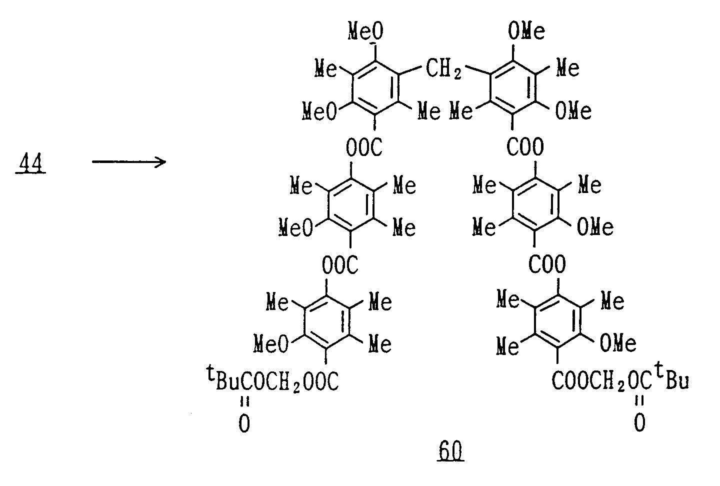

### Synthesis of 60

Compound 44 (100 mg, 83.2 µM) was dissolved in acetone (1 ml), and to the solution was added pivaloyloxymethyl iodide (tBuCOOCH₂I: 48 mg, 83.2 µM x 2.4) dissolved in 3 ml of acetone and then anhydrous K₂CO₃ (40 mg, 83.2 µM x 3.5) was added thereto, and the resultant mixture was stirred at room temperature for two and a half days. The reaction mixture was worked up in a conventional manner, and then it was subjected to column chromatography (10 g of SiO₂, eluent: toluene - ethyl acetate (19:1) to (9:1)), and the residue was crystallized from ether - n-hexane, to yield 103 mg of 60 as colorless crystals (87%). M.p. 211-213 °C. TLC (Rf: 0.3, Developer: benzene - ethyl acetate (9:1)).
- ¹HNMR (CDCl₃):: δ 1.28 (s,18H), 2.20-2.37 (m,42H), 2.41 (s,6H), 3.62 (s,6H), 3.74-3.88 (m,18H), 4.27 (s,2H), 6.01 (s,4H)
- IR (Nujol):: 1751, 1570, 1150, 983 cm⁻¹

| Elementary Analysis (for C₇₉H₉₆O₂₄·H₂O) | | |
|---|---|---|
| Theory: | C,65.55; | H,6.82 (%) |
| Found : | C,65.58; | H,6.63 (%) |

### Example 14

### Bis[5-[4'-(4''-carboxy-3''-hydroxy-2'',5'',6''-trimethylphenoxycarbonyl)-3'-hydroxy-2',5',6'-trimethylphenoxycarbonyl]-2,4-dihydroxy-3,6-dimethylphenyl] methane

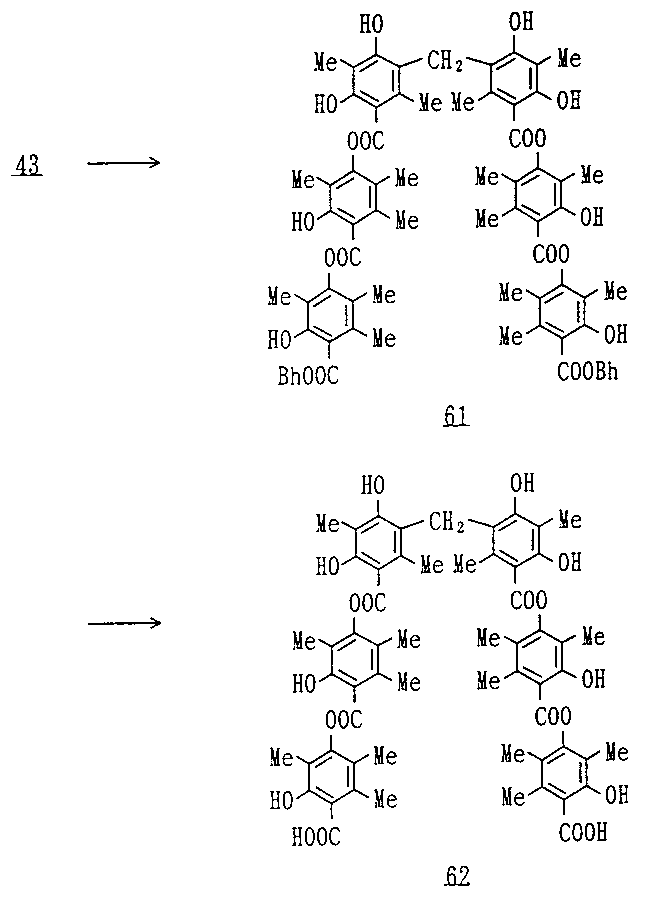

### [Step 1] 43→61

A mixture containing the compound 43 (500 mg, 326 µM), BBr₃ (371 µl, 326 µM x 12), and CH₂Cl₂ (15 ml) was stirred at room temperature for 6 hours, and water was added thereto, and then the resultant mixture was distributed between ethyl acetate and brine. The organic phase was washed with brine three times, dried, and concentrated in vacuo. The residue was dissolved in ethyl acetate (6 ml), and the solution was subjected to benzhydrylation in a procedure similar to that of Step 5 in Preparation 1, to yield the crude compound 61. The compound was subjected to column chromatography (Lober (Merck: B), eluent: toluene - ethyl acetate (1:0) to (0:1)), and the residure was powdered in ethyl - n-hexane to yield 162 mg of 61 as a colorless powder (35%). TLC (Rf: 0.5, Developer: benzene - ethyl acetate (9:1)).
- ¹HNMR (CDCl₃):: δ 2.00-2.20 (m,30H), 2.55 (s,6H), 2.67 (s,6H), 2.74 (s,6H), 4.17 (s,2H), 6.31 (s,2H), 7.20 (s,2H), 7.28-7.48 (m,20H), 11.04 (s,2H), 11.33 (s,2H), 11.56 (s,2H)

### [Step 2] 61→62

Compound 61 (61 mg, 106 µM) was subjected to hydrogenation in a procedure similar to that of Step 4 in Preparation 1, to yield 62. The compound was crystallized from ether to yield 49 mg of 62 as colorless crystals (43%). M.p. (dec.); 162-220 °C. TLC (Rf: 0.2, Developer: ethyl acetate - acetic acid (1%)).
¹HNMR (DMSO): δ 1.96-2.40 (m,48H), 4.09 (s,2H), 9.43 (s,2H), 9.90 (s,2H)
IR(Nujol): 3700-2560, 1670, 1455, 1155 cm⁻¹.

### Example 15-17

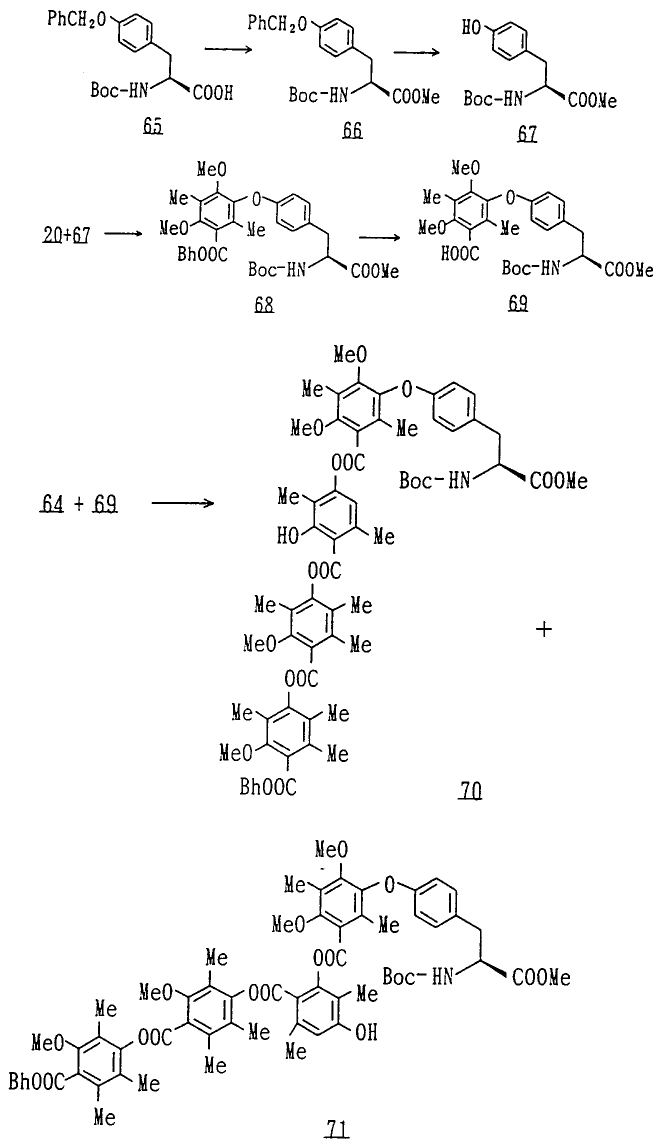

### [Step 1] Synthesis of the compound 67

Diazomethane gas was generated by stirring a solution of N-methyl nitrosourea (10 g, 20.2 mM x 4.8) in ether and an aqueous solution of potassium hydroxide, and the gas was trapped in ice-cooled ether, to make a solution of diazomethane in ether. Then, the ether solution was added to a methanol solution of N-t-Boc-O-benzyl-L-tyrosine 65 (7.5 g, 20.2 mM), until the solution developed a yellow color. Acetic acid was added thereto until the solution became colorless in order to decompose excess diazomethane. The material was concentrated in vacuo to yield 66. The compound was dissolved in methanol, and it was subjected to hydrogenation in a procedure similar to that of Step 4 in Preparation 1, to yield the crude compound 67. The compound was recrystallized from ether - n-hexane to yield 5.88 g of 67 as colorless crystals (99%). M.p. 100-101 °C. TLC (Rf: 0.3, Developer: benzene - ethyl acetate (4:1)).
- ¹HNMR (CDCl₃):: δ 1.42 (s,9H), 2.94-3.06 (m,2H), 3.72 (s,3H), 4.26-4.61 (m,1H), 4.92-5.06 (m,1H), 5.42 (s,1H), 6.74 (d,J=8.2Hz,2H), 6.98 (d,J=8.2Hz,2H)
- IR (Nujol):: 3399, 1716, 1690, 1519 cm⁻¹
[α]^{25.0}_{D}= +49.1 ± 0.9° (CHCl₃, C=1.008%)

| Elementary Analysis (for C₁₅H₂₁NO₅·0.4H₂O) | | | |
|---|---|---|---|
| Theory: | C,59.55; | H,7.26; | N,4.63 (%) |
| Found : | C,59.55; | H,6.88; | N,4.71 (%) |

### [Step 2] 20+67→68

Compound 20 (2.56 g, 5.61 mM) and 67 (1.66 g, 5.61 mM) were treated in a procedure similar to that of Step 2 in Example 1, to yield the crude compound 68. The compound was subjected to column chromatography (75 g of SiO₂, eluent: n-hexane - ethyl acetate (9:1) to (4:1)), to yield 226 mg of 68 (6%) as a colorless foam. TLC (Rf: 0.2, Developer: n-hexane - ethyl acetate (2:1)).
- ¹HNMR (CDCl₃):: δ 1.41 (s,9H), 1.95 (s,3H), 2.18 (s,3H), 2.94-3.04 (m,2H), 3.58 (s,3H), 3.70 (s,3H), 3.71 (s,3H), 4.24-4.62 (m,1H), 4.89-5.01 (m,1H), 6.72 (d,J=8.4Hz,2H), 7.00 (d,J=8.4Hz,2H), 7.17 (s,1H), 7.26-7.48 (m,10H)
- IR (Nujol):: 3370, 1720, 1169 cm⁻¹
[α]^{24.0}_{D}=+21.4 ± 0.6° (CHCl₃, C=1.002%)

| Elementary Analysis (for C₃₉H₄₃NO₉) | | | |
|---|---|---|---|
| Theory: | C,69.94; | H,6.47; | N,2.09 (%) |
| Found : | C,69.85; | H,6.44; | N,2.17 (%) |

### [Step 3] 68→69

Compound 68 (198 mg, 296 µM) was subjected to hydrogenation in a procedure similar to that of Step 4 in Preparation 1, to yield 149 mg of 69 (100%) as colorless foam. TLC (Rf: 0.1, Developer: benzene - ethyl acetate (4:1)).
- ¹HNMR (CDCl₃):: δ 1.41 (s,9H), 2.22 (s,3H), 2.23 (s,3H), 2.94-3.09 (m,2H), 3.71 (s,3H), 3.76 (s,3H), 3.86 (s,3H), 4.48-4.64 (m,1H), 4.94-5.06 (m,1H), 6.72 (d,J=8.6Hz,2H), 7.02 (d,J=8.6Hz,2H)

### [Step 4] 64+69→70+71

Compound 64 (434 mg, 296 µM x 2) and 69 (149 mg, 296 µM) were treated in a procedure similar to that of Step 4 in Preparation 2, to yield a mixture comprising 70 and 71. The mixture was subjected to column chromatography (15 g of SiO₂, eluent: toluene - ethyl acetate (2%) to (28%)), to yield 70 (145 mg, 40%) and 71 (118 mg, 33%).

Compound 70: Colorless glassy material. TLC (Rf: 0.5, Developer: benzene - ethyl acetate (9:1)).
- ¹HNMR (CDCl₃):: δ 1.42 (s,9H), 2.05-2.45 (m,27H), 2.76 (s,3H), 3.57 (s,3H), 3.72 (s,3H), 3.78 (s,3H), 3.84 (s,3H), 3.91 (s,3H), 4.52-4.64 (m,1H), 4.93-5.05 (m,1H), 6.70 (s,1H), 6.78 (d,J=8.6Hz,2H), 7.05 (d,J=8.6Hz,2H), 7.20 (s,1H), 7.25-7.50 (m,10H), 11.81 (s,1H)

Compound 71: Colorless glassy material. TLC (Rf: 0.1, Developer: benzene - ethyl acetate (9:1)).
- ¹HNMR (CDCl₃):: δ 1.90-2.38 (m,27H), 2.50 (s,3H), 2.88-3.01 (m,2H), 3.57 (s,3H), 3.65 (s,3H), 3.73 (s,3H), 3.74 (s,3H), 3.86 (s,3H), 4.46-4.58 (m,1H), 4.90-5.02 (m,1H), 5.71 (s,1H), 6.61 (d,J=8.4Hz,2H), 6.70 (s,1H), 6.98 (d,J=8.4Hz,2H), 7.20 (s,1H), 7.28-7.50 (m,10H)

### Example 18

### [5-[4'-[4''-(4'''-Carboxy-3'''-methoxy-2''',5''',6'''-trimethylphenoxycarbony)-3''-methoxy-2'',5'',6''-trimethylphenoxycarbony]-3'-hydroxy-2',5'-dimethylphenoxycarbony]-2,4-dimethoxy-3,6-dimethylphenyl] [4''''-(2'''''-amino-2'''''-carboxyethyl)phenyl] ether trifluoroacetate

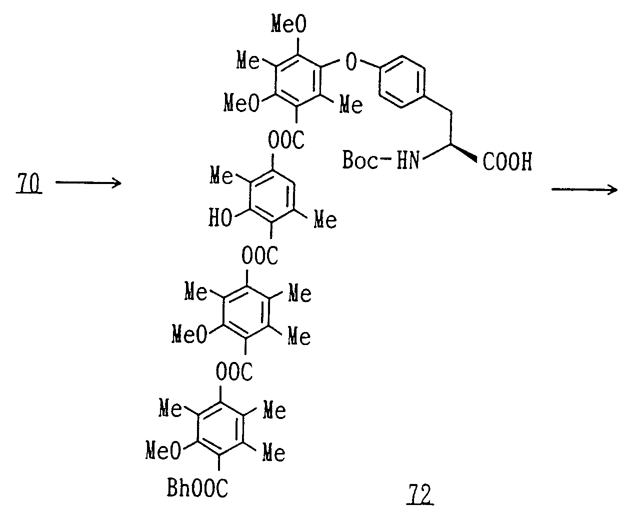

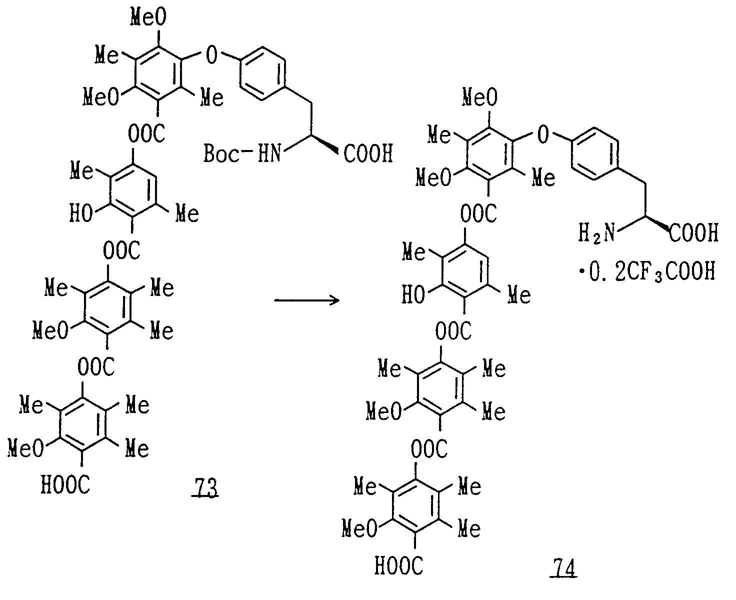

### [Step 1] 70→73

Compound 70 (128 mg, 105 µM) was hydrolyzed in a procedure similar to that of Step 3 in Preparation 1, to yield 122 mg of 72 (96%) as a colorless oil. TLC (Rf: 0.4, Developer: ethyl acetate - acetic acid (1%)). The compound was subjected to hydrogenation in a procedure similar to that of Step 4 in Preparation 1, to yield 105 mg of 73 (100%) as a colorless powder. TLC (Rf: 0.3, Developer: ethyl acetate - acetic acid (1%)).
- ¹HNMR (CDCl₃):: δ 2.10-2.46 (m,27H), 2.76 (s,3H), 3.03-3.08 (m,2H), 3.74-3.92 (m,12H), 4.52-4.68 (m,1H), 4.92-5.02 (m,1H), 6.70 (s,1H), 6.80 (d,J=8.6Hz,2H), 7.13 (d,J=8.6Hz,2H), 11.80 (s,1H)

### [Step 2]73→74

A mixture containing 73 (109 mg, 105 µM), trifluoroacetic acid (1.2 ml), and methylene chloride (2 ml) was stirred at room temperature for two hours, and the resultant mixture was concentrated directly in vacuo. The residue was dissolved in ethyl acetate, and the solution was again concentrated in vacuo. The residue was powdered in ethyl acetate - ether to yield 64 mg of 74 (58%) as a colorless powder.
- ¹HNMR (DMSO):: δ 2.10-2.58 (m,30H), 2.72-2.92 (m,2H), 3.73 (m,6H), 3.78 (s,3H), 3.84 (s,3H), 6.74 (d,J=8.6Hz,2H), 6.80 (s,1H), 7.22 (d,J=8.6Hz,2H)
- IR (Nujol):: 3680-2280, 1750, 1700, 1665, 1610, 1160, 1140, 1080 cm⁻¹
[α]^{26.0}_{D}=-12.1 ± 0.8 ° (DMSO, C=0.644%)

| Elementary Analysis (for C₅₁H₅₅NO₁₆·0.2CF₃COOH) | | | | |
|---|---|---|---|---|
| Theory: | C,64.26; | H,5.79; | N,1.49; | F,1.19 (%) |
| Found : | C,64.18; | H,5.89; | N,1.49; | F,0.95 (%) |

### Example 19

### [5-[6'-[4''-(4'''-Carboxy-3'''-methoxy-2''',5''',6'''-trimethylphenoxycarbony)-3''-methoxy-2'',5'',6''-trimethylphenoxycarbony]-3'-hydroxy-2',5'-dimethylphenoxycarbony]-2,4-dimethoxy-3,6-dimethylphenyl] [4''''-(2'''''-amino-2'''''-carboxyethyl)phenyl] ether trifluoroacetate

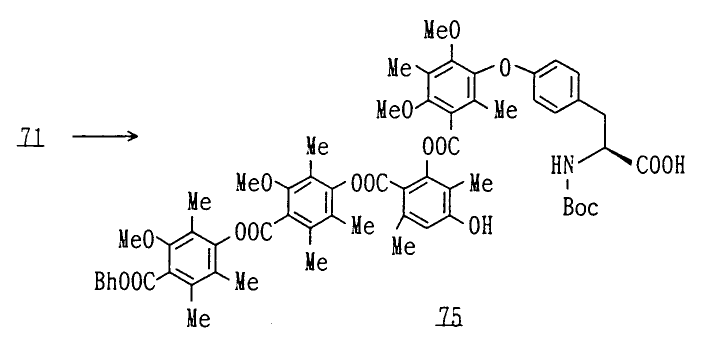

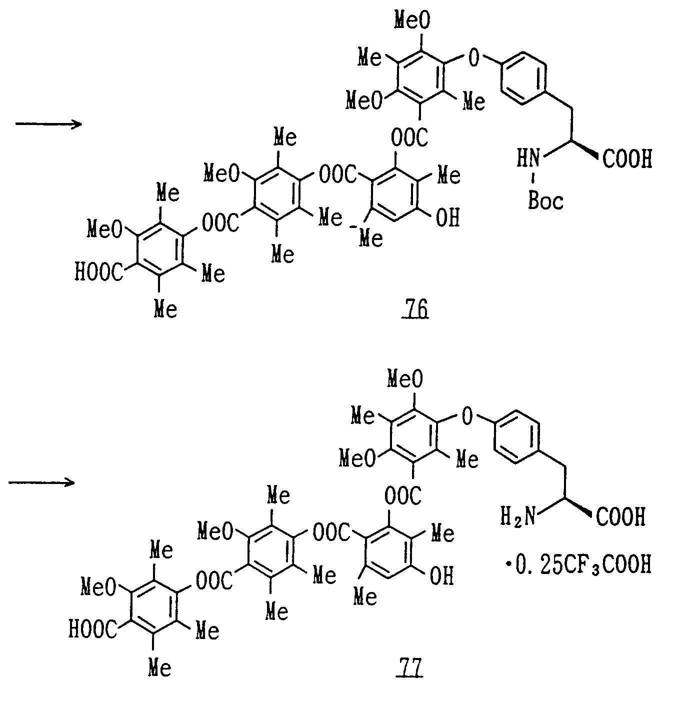

### [Step 1] 71→75

Compound 71 (110 mg, 90.3 µM) was hydrolyzed in a procedure similar to that of Step 3 in Preparation 1, to yield 100 mg of 75 (92%) as a colorless oil. TLC (Rf: 0.5, Developer: ethyl acetate - acetic acid (1%)).
- ¹HNMR (CDCl₃):: δ 1.90-2.50 (m,30H), 2.78-3.02 (m,2H), 3.56 (s,3H), 3.73 (s,3H), 3.74 (s,3H), 3.87 (s,3H), 4.40-4.54 (m,1H), 4.90-5.00 (m,1H), 6.56 (d,J=8.6Hz,2H), 6.68 (s,1H), 7.00 (d,J=8.6Hz,2H), 7.21 (s,1H), 7.28-7.50 (m,10H)

### [Step 2] 75→76

Compound 75 (100 mg, 83.0 µM) was subjected to hydrogenation in a procedure similar to that of Step 4 in Preparation 1, to yield 70 mg of 76 (81%) as a colorless powder. TLC (Rf: 0.3, Developer: ethyl acetate - acetic acid (1%)).
- ¹HNMR (d₆-acetone):: δ 1.33 (s,9H), 1.92-2.05 (m,9H), 2.18-2.40 (m,18H), 2.49 (s,3H), 2.97-3.17 (m,2H), 3.75 (s,3H), 3.78 (s,3H), 3.81 (s,3H), 3.93 (s,3H), 4.24-4.46 (m,1H), 5.91-6.02 (m,1H), 6.63 (d,J=8.6Hz,2H), 6.88 (s,1H), 7.18 (d,J=8.6Hz,2H), 7.25 (s,1H)
- IR (Nujol):: 3680-2260, 3340, 1730, 1158 cm⁻¹

### [Step 3] 76→77

Compound 76 (70 mg, 67.4 µM) was deprotected in a procedure similar to that of Step 2 in Example 18, to yield 52 mg of 77 (73%) as a colorless and hygroscopic powder. (Rf: 0.5, Developer: ethyl acetate - acetic acid - water (8:1:1)).
IR (Nujol): 3690-2400, 3400, 1735, 1276, 1158 cm⁻¹
[α]^{26.0}_{D}=-9.3 ± 1.0° (DMSO, C=0.515%)

| Elementary Analysis (for (C₅₁H₅₅NO₁₆·0.25CF₃COOH·3.5H₂O) | | | | |
|---|---|---|---|---|
| Theory: | C,60.77; | H,5.99; | N,1.34; | F,1.36 (%) |
| Found : | C,60.85; | H,5.80; | N,1.55; | F,1.42 (%) |

### Example 20

### Bis[3-[4'-[(4''-carboxy-3''-methoxy-2'',5'',6''-trimethyl)phenoxycarbony]-3'-methoxy-2',5',6'-trimethyl]-phenoxycarbony-4-methoxyphenyl] sulfide

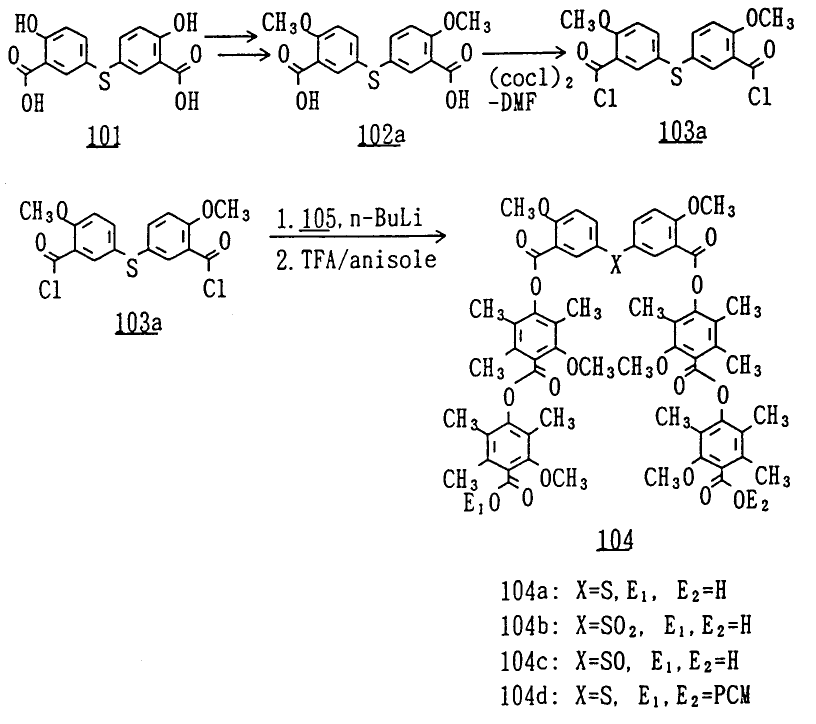

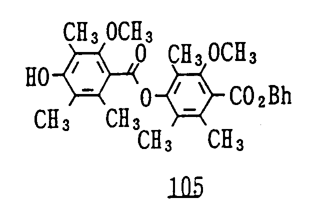

### [Step 1]

5,5'-Thio-disalicylic acid (6.62g, 0.022 mol) was dissolved in 300 ml of acetone, and 11.0 g of MeSO₄ (0.087 mol) and 15.2 g of K₂CO₃ (0.11 mol) were added thereto, and then the mixture was heated under reflux with stirring for seven hours. After cooling, 400 ml of ice-water was added to the mixture. Then, the resultant mixture was extracted with chloroform (200 ml x 2), and the organic phase was dried (Na₂SO₄), and then evaporated to dryness in vacuo, to yield 8.22 g of the residue as an oil.

The oil (8.22 g), which was the crude dimethyl ester, was dissolved in 300 ml of methanol, and 50 ml of 1 M KOH solution in water was added thereto, and then the mixture was heated under reflux for three hours. The mixture was cooled to room temperature, and then the mixture was neutralized by adding 3.3 g of acetic acid thereto. The resultant mixuture was evaporated to dryness in vacuo, and the residue was distributed between chloroform and water. The organic phase was washed with water, dried (Na₂SO₄), and then concentrated in vacuo to yield 7.7 g of the solid. The solid was washed with a small amount of ether to yield 5.78 g of 102a (76%). M.p. 158-159 °C (chlorform - n-hexane).

| Elementary Analysis (for C₁₆H₁₄O₆S) | | | |
|---|---|---|---|
| Theory (%): | C;57.34, | H;4.15, | S;9.39 |
| Found (%) : | C;57.47, | H;4.22, | S;9.59 |

### [Step 2]

The compound 102a (58.7 mg, 0.18 mmol) obtained above was dissolved in 2 ml of anhydrous methylene chloride, and to the solution was added 0.16 ml of oxalyl chloride (1.8 mmol) dropwise at room temperature, followed addition of two drops of a 10% solution of dimethylformamide in methylene chloride. The mixture was stirred at room temperature for one hour, and then at 40-50°C (temperature of an oil bath) for an additional hour.

The reaction mixture was evaporated to dryness in vacuo, and the residue was dissolved in 4 ml of anhydrous tetrahydrofuran, and the solution was evaporated to dryness in vacuo. The remaining excess of the reagents was removed by repeating the procedure twice, and then the residue was dissolved in 3 ml of tetrahydrofuran to yield a solution of 103a in tetrahydrofuran.

Compound 105 (200 mg, 0.35 mmol) was dissolved in 5 ml of tetrahydrofuran, and 0.23 ml of a 1.55 M solution of n-butyl lithium in n-hexane was added slowly thereto dropwise at - 78 °C, and then the mixture was stirred at - 78 °C for one hour. Then, to the solution was added slowly the solution of 103a in tetrahydrofuran obtained above dropwise at - 78°C, and the mixture was stirred at - 78 °C for 30 minutes. The reaction mixture was allowed to warm to room temperature with stirring over 1.5 hours. The mixture was evaporated to dryness in vacuo, and the residue was distributed between 1 N hydrochloric acid and ethyl acetate, and the organic phase was washed with an aqueous saturated sodium bicarbonate solution and a saturated brine, dried (Na₂SO₄), and then concentrated in vacuo to yield 0.28 g of a colorless oil. The oil was purified by preparative thin layer chromatography (KGF (Merck), Developer: ethyl acetate : n-hexane = 1:2) to yield 138 mg of the benzhydryl ester of 104a (54.7%).
- NMR (CDCl₃):: 2.08, 2.13, 2.17, 2.20, 2.25, 2.38 (CH₃, each s), 3.57, 3.81, 3.96 (OCH₃, each s), 7.02-8.09 (aromatic H, multiplet)

The dibenzhydrol ester of 104a (138 mg, 0.1 mmol) was dissolved in 5 ml of methylene chloride, and the solution was cooled to 0 °C, and a solution of anisole (46 mg, 0.43 mmol) and trifluoroacetic acid (110 mg) in 1 ml of methylene chloride was added to the solution. After the resultant solution was stirred at 0 °C for five hours, the reaction mixture was allowed to warm to room temperature, and evaporated to dryness to yield 181 mg of a colored sticky oil. The oil was purified by preparative thin layer chromatography (KGF (Merck), Developer: chloroform - methanol = 3:1) to yield 68 mg of 104a (64%). M.p. 278-280 °C (dec.) (recrystallized from 80% aqueous ethanol).

| Elementary Analysis (for C₆₀H₆₂O₁₈S·¹/₂H₂O) | | | |
|---|---|---|---|
| Theory (%): | C;64.96, | H;5.76, | S;2.79 |
| Found (%) : | C;64.80, | H;5.71, | S;2.88 |

- NMR (CDCl₃):: 2.14, 2.18, 2.25, 2.29, 2.36, 2.40 (CH₃, each s), 3.83, 3.86, 3.97 (OCH₃, each s), 8.095 (aromatic H,d,J=2Hz), 7.60 (aromatic H,dd,J₁=2,J₂=8.8Hz), 7.05(aromatic H,d,J=8.8Hz)

### Example 21

### Bis[3-[4'-[(4''-carboxy-3''-methoxy-2'',5'',6''-trimethyl)phenoxycarbony]-3'-methoxy-2',5',6'-trimethyl]-phenoxycarbony-4-methoxyphenyl] sulfone

The dibenzhydryl ester of 104a (200 mg, 0.14 mmol) was dissolved in 5 ml of chloroform, and to the solution was added 120 mg (0.56 mmol) of m-chloro-benzoic acid (80%) under cooling with ice-water, and the mixture was stirred for 4.5 hours.

The reaction mixture was washed with an aqueous 5% sodium thiosulfate solution, an aqueous saturated sodium bicarbonate, and water, successively, then dried, and the solvent was evaporated in vacuo to yield 260 mg of the crude sulfone dibenzhydryl ester. The dibenzhydryl ester of 104b (260 mg, 0.18 mmol), and 90 mg of anisole (0.83 mmol) were dissolved in 5 ml of methylene chloride, and the solution was cooled to 0 °C, and then a solution of trifluoroacetic acid (210 mg, 1.8 mmol) in 1 ml of methylene chloride was added thereto with stirring. After the solution has been stirred at 0°C for 3 hours, the reaction mixture was distributed between 1 N-HCl and ethyl acetate. The organic phase was washed with water, dried (Na₂SO₄), and then evaporated to dryness in vacuo, to yield 248 mg of the crude product. The product was recrystallized from ethanol (99%) to provide 123 mg of 104b. Yield: 80.9%. M.p. 294-295°C (dec.)

| Elementary Analysis (for C₆₀H₆₂O₂₀S·¹/₂H₂O) | | | |
|---|---|---|---|
| Theory(%): | C;63.04, | H;5.49, | S;2.81 |
| Found (%): | C;62.94, | H;5.55, | S;2.80 |

- NMR(CDCl₃):: 2.12, 2.16, 2.23, 2.27, 2.34, 2.38(CH₃, each s), 3.80, 3.85, 4.04(OCH₃, each s), 8.65(aromatic H, d, J=2.6Hz), 8.20(aromatic H, dd, J=2.2Hz, J=9.0Hz), 7.20(aromatic H, d, J=9.0Hz)

### Example 22

### Bis[3-[4'-[(4''-carboxy-3''-methoxy-2'',5'',6''-trimethyl)phenoxycarbony]-3'-methoxy-2',5',6'-trimethyl]-phenoxycarbony-4-methoxyphenyl] sulfoxide

The dibenzhydryl ester of 104a (200mg, 0.14 mmol) was dissolved in 5 ml of methylene chloride, and to the solution was added 30 mg (0.14 mmol) of m-chloro-benzoic acid (80%) under cooling with ice-water, and the mixture was stirred at 0 °C for 4.5 hours. The reaction mixture was washed with 5% aqueous sodium thiosulfate, an aqueous saturated sodium bicarbonate, and water, successively, and dried (Na₂SO₄), and then the solvent was evaporated in vacuo to yield 245 mg of the crude product. The product was purified by column chromatography (40 g of SiO₂, eluent: ethyl acetate - n-hexane (2:1)) to yield 151 mg of 104c-dibenzhydryl ester.

The dibenzhydryl ester of 104c (146 mg, 0.1 mmol), and 50 mg of anisole were dissolved in 5 ml of methylene chloride, and then 110 mg of trifluoroacetic acid in 1 ml of methylene chloride were added thereto with stirring whilst cooling with ice-water. After the solution had been stirred at 0°C for five hours, the reaction mixture was evaporated to dryness in vacuo, and the residue was distributed between 1 N HCl and ethyl acetate. The organic phase was washed with water, dried (Na₂SO₄), and the solvent was evaporated in vacuo, to yield 126 mg of the crude product. The product was recrystallized from ether - n-hexane to provide 118 mg of the crude compound 104c.

Then, the compound was further recrystallized from ethanol (99%) to provide 95 mg of 104c. M.p. 193-5°C

| Elementary Analysis (for C₆₀H₆₂O₁₉S·H₂O) | | | |
|---|---|---|---|
| Theory: | C;63.33, | H;5.72, | S;2.69 |
| Found : | C;63.37, | H;5.67, | S;2.82 |

- NMR(CDCl₃):: 2.11, 2.16, 2.23, 2.27, 2.34, 2.38(CH₃, each s), 3.81, 3.85, 4.01(OCH₃, each s), 8.35(aromatic H, d, J=2.2HZ), 7.92(aromatic H, dd, J=2.2, J'=9.0Hz), 7.21(aromatic H, d, J=9.0Hz)

### Example 23

### Bis[3-[4'-[(4''-pivaroyloxymethyloxycarbonyl-3''-methoxy-2'',5'',6''-trimethyl)phenoxycarbonyl]-3'-methoxy-2',5',6'-trimethyl]- phenoxycarbonyl-4-methoxyphenyl] sulfide

A mixture containing 500 mg (0.45 mmol) of 104a, 265 mg (1.09 mmol) of pivaroyloxymethyl iodide, 216 mg (1.56 mmol) of potassium carbonate and 25 ml of acetone was stirred at room temperature for 16 hours. To the mixture was added 200 ml of ice-water, and the resultant mixture was extracted with ethyl acetate. The organic phase was washed with water, dried (Na₂SO₄), and the solvent was evaporated in vacuo, to yield 0.65 g of the crude product. The product was recrystallized from ethyl acetate - n-hexane to provide 0.516 g of 104d. M.p. 120°C (dec.)

| Elementary Analysis (for C₇₂H₈₂O₂₂S·¹/₂ hexane) | | | |
|---|---|---|---|
| Theory(%): | C;65.69, | H;6.73, | S;2.28 |
| Found (%): | C;65.53, | H;6.53, | S;2.33 |

### Example 24

### 3-Carboxy-4-methoxyphenyl{3'-[4''-(4'''-carboxy-3'''-methoxy-2''',5''',6'''-trimethylphenoxycarbony)-3''-methoxy-2'',5'',6''-trimethylphenoxycarbonyl]-4'-methoxyphenyl]} sulfide

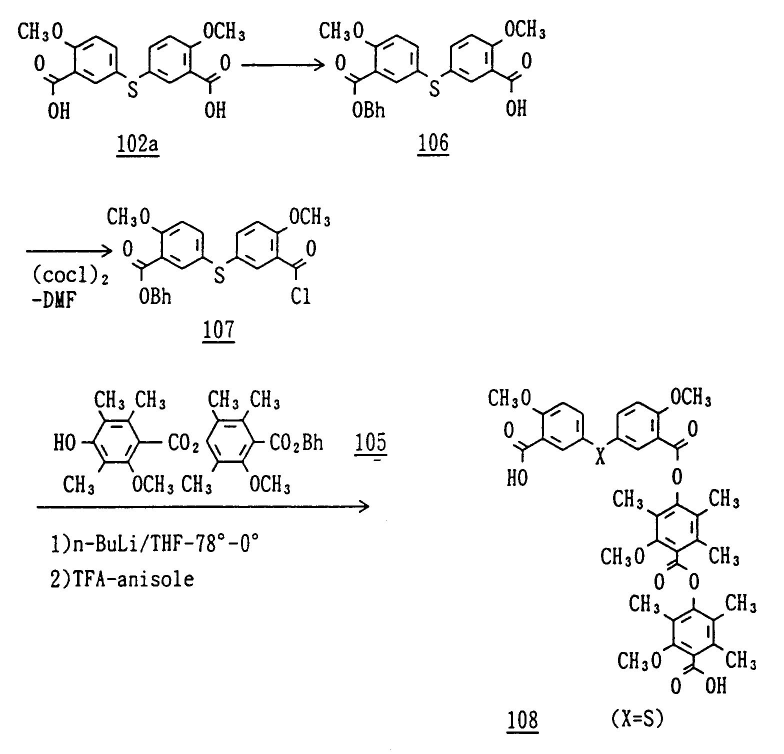

### [Step 1]

Compound 102a (1.31 g, 3.9 mmol) was dissolved in 50 ml of methylene chloride, and to the solution was added 0.8 g (4.1 mmol) of diphenyldiazomethane whilst cooling with ice-water, and the mixture was stirred for 1.8 hours. The solvent was evaporated in vacuo, and the residue was applied to column chromatography (SiO₂, 150g), and it was eluted with benzene - ethyl acetate (5:1) and then chloroform - methanol (9:1), to yield 1.06 g of 106 (54%).

### [Step 2]

Compound 106 (300 mg, 0.6 mmol) and 761 mg of oxalyl chloride (0.52 mmol) were reacted by a procedure similar to that of Step 1 in Example 20, to provide 9 ml of the solution of 107 in anhydrous tetrahydrofuran. Then, 341 mg (0.6 mmol) of 105 in 15 ml of the anhydrous tetrahydrofuran, and n-butyl lithium in hexane (0.37 ml, 1.55 M) were subjected to a condensation reaction according to Step 2 in Example 20, to yield 688 mg of the crude product. The product was purified by silica gel chromatography (40 g of SiO₂, eluent: ethyl acetate - n-hexane (1:2)), to yield 213 mg (0.2 mmol) of the dibenzhydryl ester.

The dibenzhydryl ester obtained above was dissolved in 10 ml of methylene chloride, and deprotection was conducted using 231 mg (2 mmol) of trifluoroacetic acid and 100 mg (0.92 mmol) of anisole according to the procedure of Step 2 in Example 20, to yield 123 mg of 108. M.p. 192-194 °C (recrystallized from aqueous ethanol).

| Elementary Analysis (for C₃₈H₃₈O₁₂S) | | | |
|---|---|---|---|
| Theory(%): | C;63.40, | H;5.30, | S;4.36 |
| Found (%): | C;63.50, | H;5.33, | S;4.46 |

- NMR (CDCl₃) :: 2.14, 2.19, 2.25, 2.29, 2.35, 2.39 (CH₃, each s), 3.83, 3.85, 3.97, 4.08 (OCH₃, each s), 8.08 (aromatic H,d,J=2.4Hz), 8.18 (aromatic H,d,J=2.4Hz), 7.60 (aromatic H,dd,J=2.4Hz,J'=8.8Hz), 7.55 (aromatic H,dd,J=2.4Hz,J'=8.8Hz), 7.03 (aromatic H,d,J=8.8Hz), 7.05 (aromatic H,d,J=8.8Hz)

### Example 25

### Bis[3-(4'-carboxy-3'-methoxy-2',5',6'-trimethylphenoxycarbonyl)-4-methoxyphenyl] sulfide (110b)

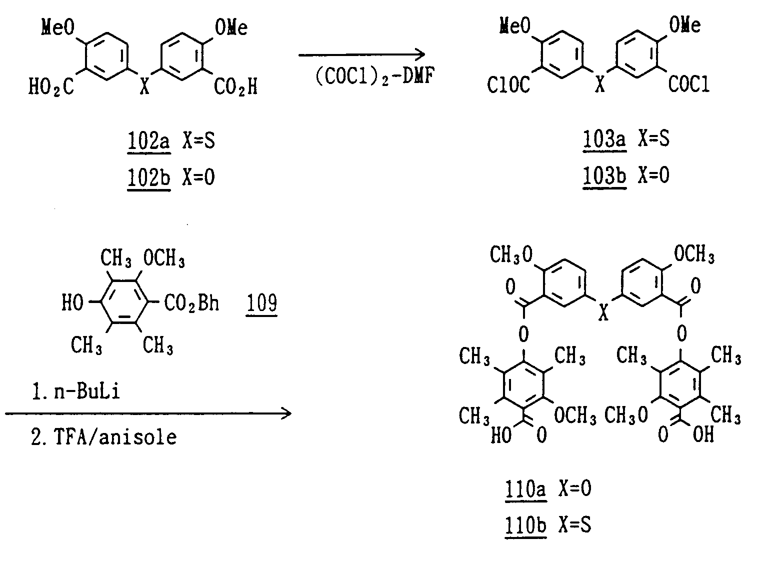

Compound 102a (150 mg, 0.45 mmol) and 0.4 ml of oxalyl chloride (4.6 mmol) were reacted in a procedure similar to that of Step 1 in Example 20, to provide 3 ml of the solution of 103a in anhydrous tetrahydrofuran. Then, 338 mg (0.9 mmol) of 109 which had been dissolved in 50 ml of the anhydrous tetrahydrofuran, and 0.56 ml of n-butyl lithium in hexane (1.55 M, 0.9 mmol) were subjected to a condensation reaction according to Step 2 in Example 20, to yield 504 mg of the composition, which was purified by silica gel chromatography (35 g of SiO₂, eluent: ethyl acetate - n-hexane (2:3)), to yield 0.464 g of the dibenzhydryl ester of 110b.

The dibenzhydryl ester (0.464 g) obtained above was dissolved in 10 ml of methylene chloride, and deprotection was conducted using 215 mg of anisole (2 mmol) and 503 mg of trifluoroacetic acid (4.4 mmol) according to the procedure of Step 2 in Example 20, to yield 227 mg of 110b. M.p. 237-239 °C (recrystallized from benzene).

| Elementary Analysis (for C₃₈H₃₈O₁₂S·¹/₂ benzene) | | | |
|---|---|---|---|
| Theory(%): | C;65.06, | H;5.51, | S;4.11 |
| Found (%): | C;64.98, | H;5.45, | S;4.23 |

NMR(CDCl₃): 2.08, 2.09, 2.32 (CH₃, each s), 3.81, 3.95 (OCH₃, each s), 8.09 (aromatic H,d,J=2.4Hz), 7.60 (aromatic H,dd,J=2.4Hz,J'=8.8Hz), 7.03 (aromatic H,d,J=8.8Hz)

### Example 26

### Bis[3-[4'-carboxy-3'-methoxy-2',5',6'-trimethylphenoxycarbonyl)-4-methoxyphenyl] ether (110a)

Compound 102b (150 mg, 0.47 mmol) and 0.41 ml of oxalyl chloride (47 mmol) were reacted in a procedure similar to that of Step 1 in Example 20, to provide 5 ml of the solution of 103b in anhydrous tetrahydrofuran. Then, 355 mg (0.94 mmol) of 109 which had been dissolved in 15 ml of the anhydrous tetrahydrofuran, and 0.59 ml of n-butyl lithium in hexane (1.55 M, 0.94 mmol) were subjected to a condensation reaction according to Step 2 in Example 20, to yield 539 mg of the composition, which was purified by silica gel chromatography (35 g of SiO₂, eluent: ethyl acetate - n-hexane (2:3)), to yield 490 mg of the dibenzhydryl ester of 110a.

The dibenzhydryl ester (490 mg, 0.47 mmol) obtained above was dissolved in 5 ml of methylene chloride, and deprotection was conducted using 228 mg (2.1 mmol) of anisole and 534 mg (4.7 mmol) of trifluoroacetic acid according to the procedure of Step 2 in Example 20, to yield 214 mg of 110a. M.p. 194-5 °C (recrystallized from aqueous ethanol).

| Elementary Analysis (for C₃₈H₃₈O₁₃·H₂O) | | |
|---|---|---|
| Theory(%): | C;63.28, | H;5.38 |
| Found (%): | C;63.33, | H;5.59 |

- NMR (CDCl₃):: 2.11, 2.12, 2.33 (CH₃, each s), 3.82, 3.95 (OCH₃, each s), 7.73 (aromatic H,d,J=3Hz), 7.27 (aromatic H,dd,J=3Hz,J'=9.2Hz), 7.06 (aromatic H,d,J=9.2Hz)

### Example 27

### Bis[3-[4'-[(4''-carboxy-3''-methoxy-2'',5'',6''-trimethylphenoxycarbonyl)-3'-methoxy-2',5',6'-trimethylphenoxycarbony]-4-methoxyphenyl] ether (120)

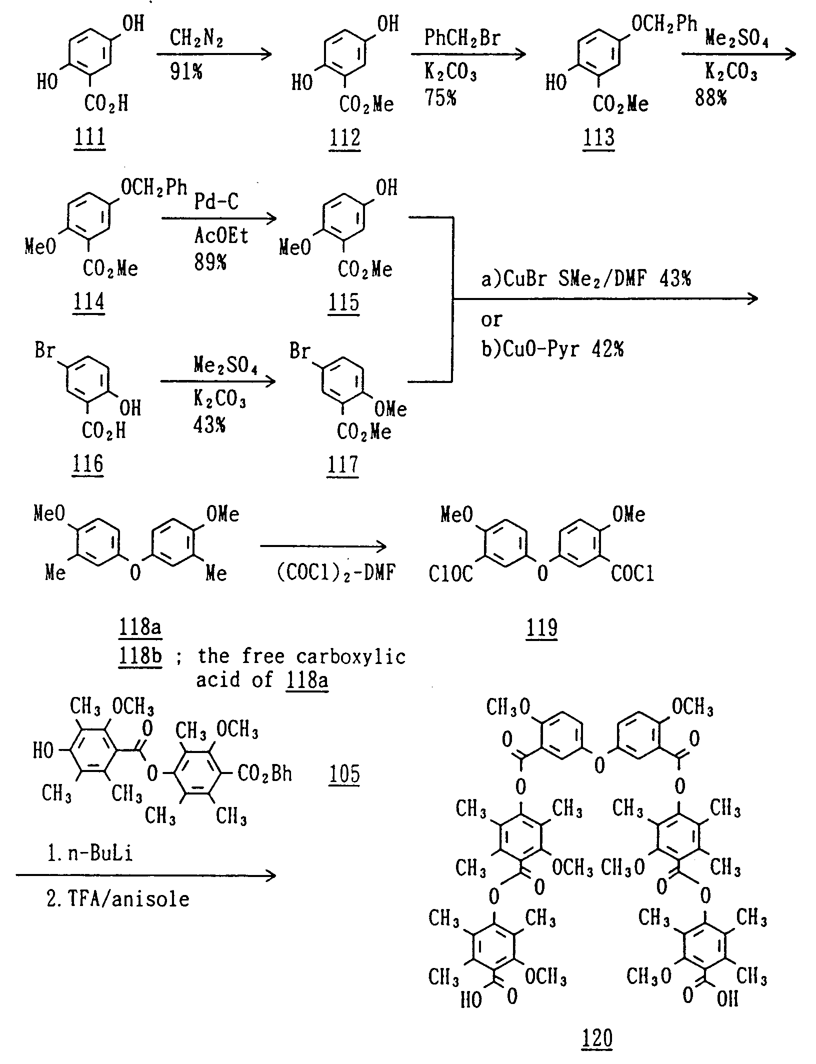

### [1] Preparation of the intermediate 118a

Compound 115 (200 mg, 1.1 mmol) was dissolved in 5 ml of DMF, and 46 mg of NaH (60 % in oil) (1.15 mmol) was added thereto, and the mixture was stirred for 1.5 hours. After 690 mg (3.36 mmol) of copper bromide (I) - dimethyl sulfide complex was added to the mixture, 270 mg of the bromide 117 (1.1 mg) which had been dissolved in 0.5 ml of DMF was added thereto, and the resultant mixutre was refluxed with stirring for 22 hours. After cooling, the reaction mixture was distributed between 1 N HCl and ethyl acetate, and the organic phase was washed with N-HCl, water, and saturated brine, successively, and then dried (Na₂SO₄). The solvent was evaporated to dryness, and the residue (383 mg) was applied to silica gel chromatography (40 g of SiO₂, eluent: ethyl acetate : hexane (2:3)), to yield 163 mg of 118a. M.p. 94-95 °C (ethyl acetate - hexane (2:3))

| Elementary Analysis (for C₁₈H₁₈O₇) | | |
|---|---|---|
| Theory(%): | C;62.26, | H;5.25 |
| Found (%): | C;62.42, | H;5.24 |

Alternatively, the compound 118a may be obtained by the following procedure.

A mixture containing 200 mg (1.1 mmol) of 115, 272 mg of 117 (1.1 mmol), 167 mg of K₂CO₃ (1.2 mmol), and 4 ml of pyridine was heated to 130 °C (oil bath) with stirring for 15 minutes, and then 87 mg of cupric oxide (II) (1.1 mmol) were added thereto, and the mixture was heating to 140 °C (oil bath) with stirring for 23 hours. After coolong, the reaction mixture was diluted with 50 ml of ether, and the resultant precipitate was filtered off, and then the filtrate was washed with the dilute hydrochloric acid, water, and saturated brine, successively, and then dried (Na₂SO₄). The solvent was evaporated to dryness, and the residue (293 mg) was subjected to silica gel chromatography, to yield 161 mg of 118a. Yield: 42.3%.

### [2] Preparation of the intermediate 118b

Compound 118a (684 mg, 2 mmol) was dissolved in 10 ml of methanol, and 5 ml of 1 M potassium hydroxide aqueous solution was added thereto, and then the mixture was heated under reflux for 5 hours. The reaction mixture was evaporated to dryness in vacuo, and the residue was distributed between 1 N HCl and ethyl acetate. After the organic phase had been washed with water and dried (Na₂SO₄), the solvent was evaporated to dryness, and then the residue (560 mg) was recrystallized from methanol, to yield 268 mg of 118b. M.p. 178-180 °C

| Elementary Analysis (for C₁₆H₁₄O₇) | | |
|---|---|---|
| Theory(%): | C;60.32, | H;4.47 |
| Found (%): | C;60.38, | H;4.43 |

### [3] Preparation of the title compound

Compound 118b (100 mg, 0.31 mmol) and 0.3 ml of oxalyl chloride (3.4 mmol) were reacted in a procedure similar to that of Step 1 in Example 20, to provide 5 ml of the solution of 119 in anhydrous tetrahydrofuran. Then, 357 mg (0.63 mmol) of 105 which had been dissolved in 10 ml of the anhydrous tetrahydrofuran, and 0.39 ml of n-butyl lithium in hexane (1.59 M, 0.62 mmol) were subjected to condensation reaction according to Step 2 in Example 20, to yield 490 mg of the crude product. The compound was purified by silica gel chromatography (45 g of SiO₂, eluent: ethyl acetate - n-hexane (2:3)), to yield 443 mg of the dibenzhydryl ester of 120.

The dibenzhydryl ester of 120 (440 mg, 0.31 mmol) obtained above was dissolved in 5 ml of methylene chloride, and deprotection was conducted using 150 mg (1.4 mmol) of anisole and 370 mg (3.2 mmol) of trifluoroacetic acid according to the procedure of Step 2 in Example 20, to yield 285 mg of 120.
M.p. 258-260 °C (recrystallized from aqueous ethanol).

| Elementary Analysis (for C₆₀H₆₂O₁₉·¹/₂EtOH) | | |
|---|---|---|
| Theory(%): | C;65.80, | H;5.75 |
| Found (%): | C;66.00, | H;5.90 |

NMR (CDCl₃): 2.16, 2.20, 2.26, 2.29, 2.36, 2.38, 2.40 (CH₃, each s), 3.83, 3.86, 3.97 (OCH₃, each s), 7.74(aromatic H,d,J=3.2Hz), 7.29(aromatic H,dd,J=3.2HZ,J'=9.2Hz), 7.08 (aromatic H,d,J=9.2Hz)

### Example 28

### Trans-1,2-bis[4'-(4''-carboxy-3''-methoxy-2'',5'',6''-trimethylphenoxycarbonyl)-3^{'}-methoxy-2',5',6'-trimethylphenoxycarbony] ethene (121a)

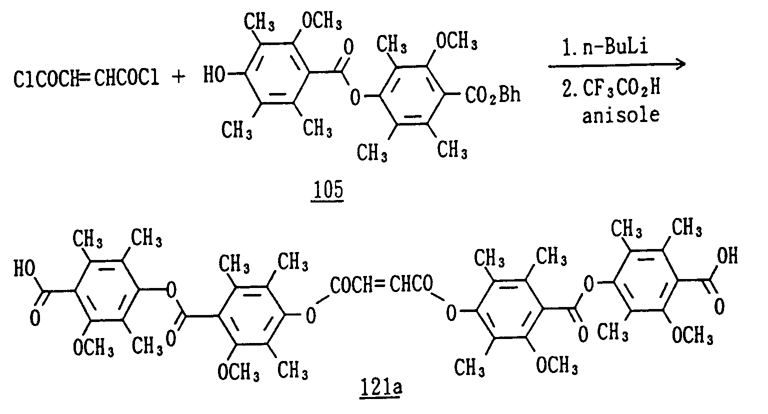

Compound 105 (2.0 g, 3.5 mmol) was dissolved in 80 ml of anhydrous tetrahydrofuran, and 2.2 ml of n-butyl lithium in hexane (1.59 M) was added dropwise slowly at - 78 °C. The mixture was stirred at -78 °C for 50 minutes, and 270 mg of fumaryl chloride (0.19 ml, 1.77 mmol) which had been dissolved in 3 ml of tetrahydrofuran was added dropwise thereto. After the mixture was stirred at - 78 °C for additional 4.5 hours, the solvent was evaporated in vacuo, and then the residue was distributed between 1 N HCl and ethyl acetate. The organic phase was washed with water, dried, and then the solvent was evaporated in vacuo, to yield 2.267 g of the crude dibenzhydryl ester.

The crude product (2.0 g) obtained above was dissolved in 50 ml of methylene chloride, and 0.82 g (7.6 mmol) of anisole and 1.87 g (16.4 mmol) of trifluoroacetic acid were added thereto at 0 °C, and the mixture was stirred at 0 °C for 3.5 hours. The solvent was evaporated in vacuo, and the residue was washed with ether to provide 1.24 g of 121a as a colorless solid. The compound was recrystallized from N,N-dimethylformamide. M.p. above 300 °C.

| Elementary Analysis (for C₄₈H₅₂O₁₆·¹/₂H₂O·¹/₂DMF) | | |
|---|---|---|
| Theory(%): | C;64.01, | H;5.91 |
| Found (%): | C;64.01, | H;6.09 |

- NMR (d₆-DMSO):: 2.45, 2.48, 2.52, 2.53, 2.53, 2.70 (CH₃, each s), 4.07, 4.11 (OCH₃, each s), 7.78 (aromatic H,s)

### Example 29

### 1,2-Bis[4'-[4''-carboxy-3''-methoxy-2'',5'',6''-trimethylphenoxycarbonyl)-3'-methoxy-2',5',6'-trimethylphenoxycarbony] ethane (122)

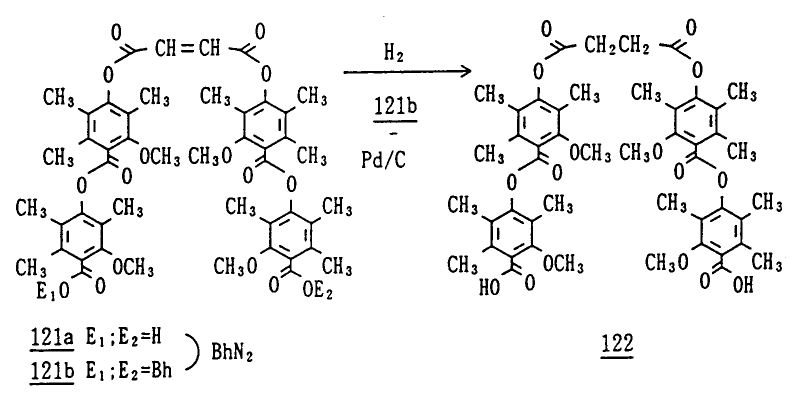

The dibenzhydryl ester 121b (100 mg) which had been obtained in Example 28 was dissolved in a mixture consisting of 5 ml of ethyl acetate and 0.5 ml of acetic acid, and the resultant mixture was reduced at atmospheric pressure using 20 mg of 10% Pd-carbon as catalyst. The catalyst was filtered off, and the precipitated crystals were dissolved in chloroform - methanol, and the solution was combined with the above filtrate. The mixture was evaporated in vacuo, and the residue was distributed between ethyl acetate and 1 N HCl, and the organic phase was washed with water, dried (Na₂SO₄), and then evaporated to dryness in vacuo to yield 92 mg of a colorless solid. The solid was recrystallized from chloroform - n-hexane to yield 42 mg of 122. M.p. > 270 °C (dec.).

| Elementary Analysis (for C₄₈H₅₄O₆) | | |
|---|---|---|
| Theory(%): | C;65.00, | H;6.14 |
| Found (%): | C;65.17, | H;6.11 |

- NMR (d₆-DMSO):: 2.04, 2.07, 2.17, 2.18, 2.19, 2.32 (CH₃, each s), 3.18 (CH₂,s), 3.72, 3.73 (OCH₃,s,6H each)

### Example 30

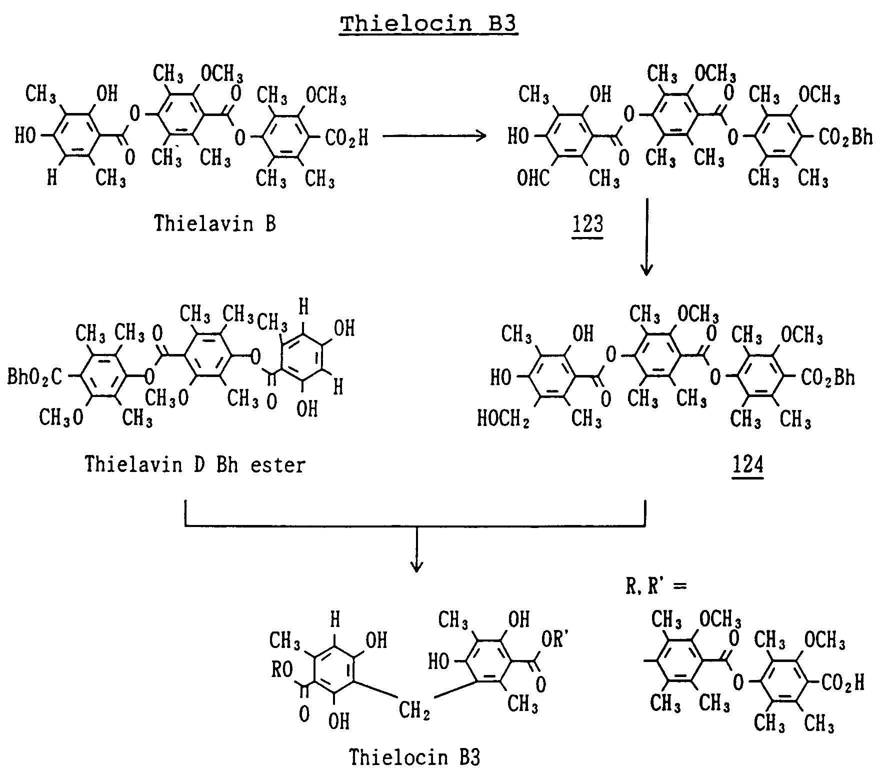

### [Step 1]

Thielavin B (5.0 g, 8.82 mmol) was dissolved in 100 ml of trifluoroacetic acid, and the solution was cooled to 0 °C. To the solution was added 1.5 g of hexamethylenetetramine (0.01 mmol), and the mixture was stirred at 0 °C for one hour, and then it was heated at 50 °C (bath temperature) with stirring for 4.5 hours. The reaction mixture was evaporated to dryness in vacuo, and to the residue were added 300 ml of water, and then the mixture was heated at 60 °C (bath temperature) with stirring for 6 hours. After cooling, the precipitated solid was filtered off, washed thoroughly with water, and then dissolved in about 200 ml of ethyl acetate. The solution was dried over Na₂SO₄, and the solvent was evaporated in vacuo to yield 5.34 g of a foam. The foam was dissolved in 100 ml of chloroform, and 1.94 g of diphenyldiazomethane was added thereto at 0 °C, and the mixture was stirred for two hours. The solvent was evaporated in vacuo, and the residue (5.84 g) was subjected to silica gel chromatography (350 g of SiO₂, eluent: ethyl acetate - n-hexane (1:4)), to yield 2.32 g of 123.

### [Step 2]

Compound 123 (2.03 g, 2.67 mmol) obtained above was dissolved in 50 ml of methanol, and 0.5 g (0.013 mol) of solid NaBH₄ was added thereto in small portions at room temperature, and after the addition was completed, the resultant mixture was stirred for an additional 1.5 hours. The reaction mixture was concentrated in vacuo, and the residue was distributed between 1 N HCl and ethyl acetate. The organic phase was washed with saturated brine, dried (Na₂SO₄), and then evaporated in vacuo to yield 2.22 g of the crude product. The product was recrystallized from a mixture consisting of ethyl acetate and n-hexane (1:3) to yield 1.75 g of 124.
- NMR (CDCl₃):: 2.09, 2.12, 2.17, 2.21, 2.25, 2.40, 2.65 (CH₃, each s), 3.57, 3.83 (OCH₃, each s), 5.03 (CH₂OH,br.S), 8.50 (s,OH), 11.65 (s,OH)

### [step 3]

Compound 124 (1.5 g, 2 mmol), and the benzhydryl ester of thielavin D (1.4 g, 2 mmol) were dissolved in 150 ml of toluene, and 0.24 ml (2 mmol) of boron trifluoride diethyl ether complex was added thereto, and the mixture was stirred at room temperature for 30 minutes. The reaction mixture was diluted with 150 ml of ethyl acetate, washed with water, and then dried (Na₂SO₄). The mixture was evaporated in vacuo, and the residue (3.61 g) was dissolved in 40 ml of methylene chloride, and to the solution were added 0.96 g of anisole and 2.2 g of trifluoroacetic acid whilst cooling with ice-water, and the mixture was stirred for one hour. The resultant mixture was left to stand overnight, and then the solvent was evaporated in vacuo. To the residue was added dilute hydrochloric acid, and the precipitated oil was collected by decantation and dissolved in ethyl acetate. The ethyl acetate phase was washed with water, dried (Na₂SO₄), and evaporated in vacuo to yield 3.79 g of the crude product. The product was purified by silica gel chromatography (eluent: chloroform - methanol - acetic acid = 500:50:25), to yield 1.234 g of the thielocin B3.

Thielocin B3: recrystallized from an aqueous ethanol, m.p. 205-207 °C (dec.)
- IR (KBr):: 3400, 1740, 1710, 1648, 1610, 1280, 1145 cm⁻¹
- NMR (CDCl₃):: 2.13, 2.18, 2.26, 2.29, 2.35, 2.41, 2.65, 2.89 (CH₃, each s), 3.85, 3.86 (OCH₃, each s), 6.37(aromatic H,s.), 11.52, 12.52 (OH, each s, disappeared by adding D₂O)

| Elementary Analysis (for C₆₂H₆₆O₂₀·2H₂O) | | |
|---|---|---|
| Theory(%): | C;64.03, | H;6.22 |
| Found (%): | C;63.80, | H;6.05 |

### Example 31

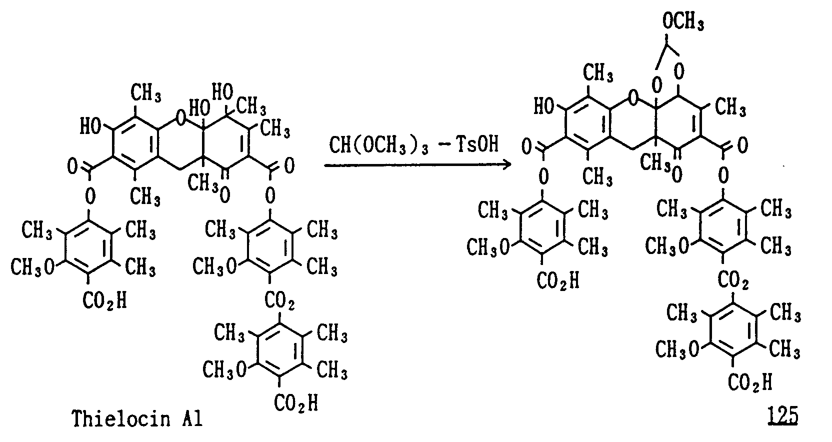

To a solution of thielocin A1 (6.5 mg) in 0.3 ml of chloroform were added trimethyl ortho-formate (1.8 ml) and p-toluene sulfonic acid (60 mg), and the mixture was left to stand at room temperature for five days. The reaction mixture was distributed between ethyl acetate and water, and the organic phase was dried (Na₂SO₄), and evaporated in vacuo. The residue was purified by thin layer chromatography (Developer: CHCl₃ : MeOH : water 62:25:4, Rf: 0.3) to yield 5.3 mg of 125. M.p. 198-200 °C. HRMS MNa⁺ bbsd m/z 1061. 3763 (Theory: 1061.3779 for C₅₆H₆₂O₁₉)

The compounds of the present invention were tested for their Phospholipase A₂ inhibitory activity by the following procedure.

1-Palmitoyl-2-[1-¹⁴C]-linoleoyl L-3-Phosphatidylethanolamine (Amersham, Inc., 59 mCi/mmol) were diluted with L-α-phosphatidylethanolamine (Sigma, Co., from egg albumin) [2,000 dpm/nmol], and the dilution was sonicated. The resultant dilution was used as a substrate. The PLA₂ (phospholipase A₂) which was used in the test was from rat platelets. The PLA₂ and the substrate preparation were added to a solution of CaCl₂ (3 mM) in Tris-buffer (0.1 M, pH 7.4), and the mixture was allowed to react at 37 °C for 20 minutes. Then, the reaction was terminated by adding 1.25 ml of Dole's reagent to the reaction mixture and stirring immediately the resultant mixture. To the mixture was added 0.5 ml of distilled water and 0.8 ml of n-heptane, and the mixture was stirred, centrifuged, and the supernatant was taken into another tube. To this supernatant were added an additional 0.8 ml of n-heptane and silica gel, and the mixture was stirred, centrifuged, and the supernatant was taken into vials. Toluene cocktail was added to the vials. The amount of free fatty acid released by PLA₂ was determined using a liquid scintillation counter.

Inhibitory activity (%) was estimated by the formula: [(DPM value at the addition of the inhibitor - DPM value without PLA₂) / (DPM value with only PLA₂ - DPM value without PLA₂)] × 100.

The results are shown in the following Table 1.

**Table 1**

| PLA₂ inhibitory activity [IC50(µM)] | |
|---|---|
| Compound No. | Rat platelets |
| 8 | > 330 |
| 9 | > 480 |
| 52 | 0.16 |
| 50 | 4.6 |
| 44 | 0.13 |
| 48 | 3.0 |
| 54 | 0.88 |
| 59 | 1.45 |
| 56 | 0.035 |
| 62 | 0.95 |
| 74 | 0.070 |
| 77 | 0.34 |
| 104a | 0.049 |
| 108 | 2.1 |
| 104b | 0.050 |
| 121 | 0.33 |
| 120 | 0.042 |
| 110b | 2.5 |
| 104c | 0.12 |
| 110a | 3.4 |
| 122 | 0.04 |

## Claims

1. Thielocin derivatives of the formula: 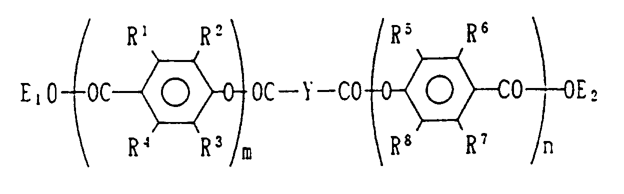 wherein R¹, R², R³, R⁴, R⁵, R⁶, R⁷, and R⁸ are independently hydrogen, lower alkyl, lower alkoxy, hydroxy, or halogen;
E₁ and E₂ are independently hydrogen, or an ester residue;
m and n are independently an integer of 0 to 4;
-Y- is a bivalent group which is selected from the group consisting of the following radicals:
-CH₂CH₂- , -CH=CH- , 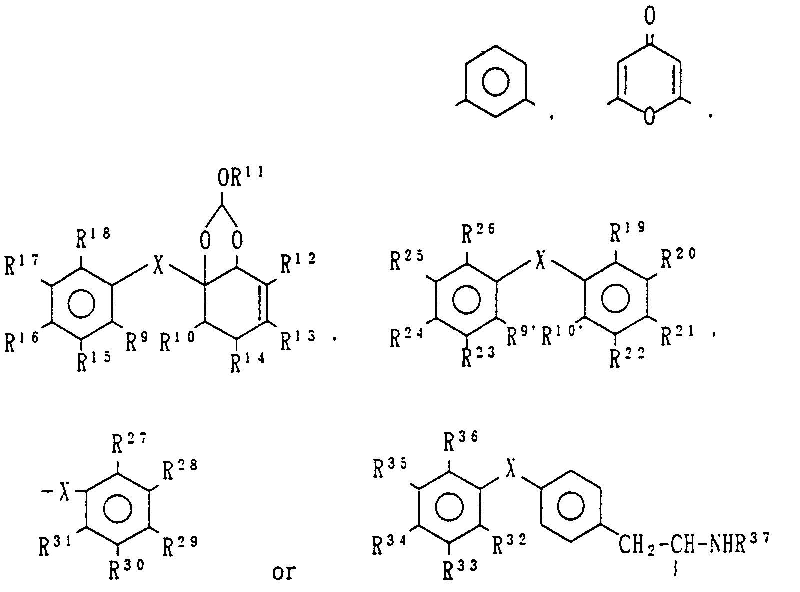 provided that m + n is not zero, when Y is -CH₂CH₂-, -CH=CH-, 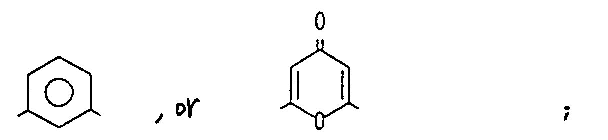 wherein X is a single bond, CH₂, O, S, SO, or SO₂;
R⁹ and R¹⁰ are independently a single bond, hydrogen, lower alkyl, lower alkoxy, hydroxy, or halogen, or R⁹ and R¹⁰ may be combined together to form methylene, ether, sulfide, sulfinyl, or sulfone;
R^{9'} and R^{10'} are independently a single bond, hydrogen, lower alkyl, lower alkoxy, hydroxy, or halogen, or R^{9'} and R^{10'} may be combined together to form methylene, ether, sulfide, sulfinyl, or sulfone;
R¹¹ is hydrogen or lower alkyl;
R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, and R³¹ are independently a single bond, hydrogen, lower alkyl, lower alkoxy, hydroxy, or halogen,
provided that one of R⁹, R¹⁵, R¹⁶, R¹⁷ and R¹⁸, one of R¹⁰, R¹², R¹³ and R¹⁴, one of R^{9'}, R²³, R²⁴, R²⁵ and R²⁶, one of R^{10'}, R¹⁹, R²⁰, R²¹ and R²², and one of R²⁷, R²⁸, R²⁹, R³⁰ and R³¹ are a single bond;
R³², R³³, R³⁴, R³⁵, and R³⁶ are independently hydrogen, lower alkyl, lower alkoxy, hydroxy, halogen, or Z, and R³⁷ is hydrogen or an amino-protecting group, wherein Z is a single bond or a bivalent group of the formula: 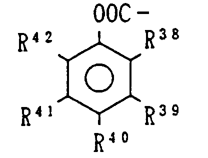 R³⁸, R³⁹, R⁴⁰, R⁴¹, and R⁴² are independently a single bond, hydrogen, lower alkyl, lower alkoxy, hydroxy, or halogen,
provided that one of R³², R³³, R³⁴, R³⁵ and R³⁶ is always Z, and when Z is not a single bond, one of R³⁸, R³⁹, R⁴⁰, R⁴¹ and R⁴² is a single bond, or when two or more of R³², R³³, R³⁴, R³⁵ and R³⁶ are Z, only one of Zs and R³⁸, R³⁹, R⁴⁰, R⁴¹ and R⁴² is a single bond;
or the salts thereof.

2. A pharmaceutical preparation for oral or external use which preparation comprises a thielocin derivative as claimed in claim 1 in association with a pharmaceutical carrier.

3. A pharmaceutical preparation as claimed in claim 2 comprising daily dosage of 0.1 mg to 500 mg of a thielocin derivative according to claim 1.

4. A pharmaceutical preparation as claimed in claim 3 suitable for oral administration and comprising a daily dosage of from 0.5 mg to 100 mg of a thielocin derivative according to claim 1.

5. A pharmaceutical preparation as claimed in claim 3 or claim 4 in unit dosage form comprising from one to five portions making up said daily dosage.

6. Thielocin derivatives as claimed in claim 1, for use as phospholipase A₂ inhibitors.

## Patentansprüche

1. Thielocinderivate der Formel: 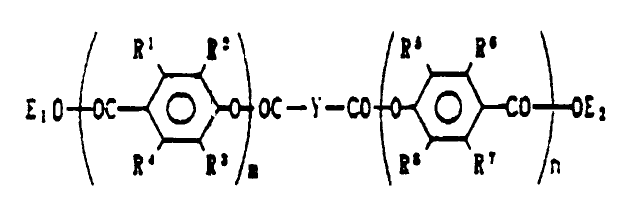 in der R¹, R², R³, R⁴, R⁵, R⁶, R⁷ und R⁸ unabhängig voneinander Wasserstoffatome, Niederalkyl-, Niederalkoxy-, Hydroxygruppen oder Halogenatome sind;
E₁ und E₂ unabhängig voneinander Wasserstoffatome oder Esterreste sind;
m und n unabhängig voneinander ganze Zahlen von 0 bis 4 sind;
-Y- ein zweiwertiger Rest ist, ausgewählt aus den folgenden Resten:
-CH₂CH₂- , -CH=CH- , 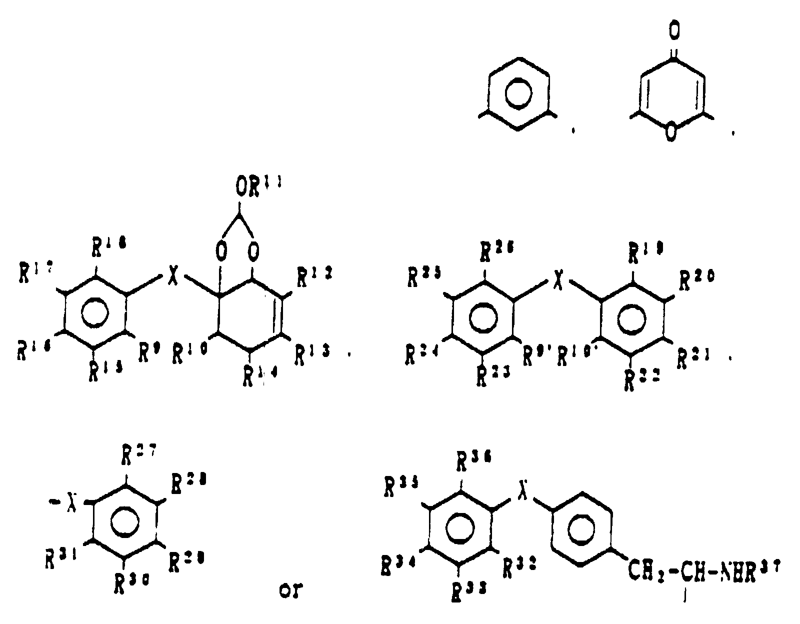 mit der Maßgabe, daß m + n ungleich null ist, wenn Y -CH₂CH₂-, -CH=CH-, 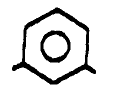 oder 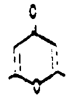 ist;
wobei X eine Einfachbindung, CH₂, O, S, SO oder SO₂ ist,
R⁹ und R¹⁰ unabhängig voneinander Einfachbindungen, Wasserstoffatome, Niederalkyl-, Niederalkoxy-, Hydroxygruppen oder Halogenatome sind oder R⁹ und R¹⁰ miteinander zu einer Methylen-, Ether-, Sulfid-, Sulfinyl- oder Sulfongruppe kombiniert sein können;
R^{9'} und R^{10'} unabhängig voneinander Einfachbindungen, Wasserstoffatome, Niederalkyl-, Niederalkoxy-, Hydroxygruppen oder Halogenatome sind oder R^{9'} und R^{10'} miteinander zu einer Methylen-, Ether-, Sulfid-, Sulfinyl- oder Sulfongruppe kombiniert sein können;
R¹¹ ein Wasserstoffatom oder ein Niederalkylrest ist;
R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, und R³¹ unabhängig voneinander Einfachbindungen, Wasserstoffatome, Niederalkyl-, Niederalkoxy-, Hydroxygruppen oder Halogenatome sind,
mit der Maßgabe, daß einer der Reste R⁹, R¹⁵, R¹⁶, R¹⁷ und R¹⁸, einer der Reste R¹⁰, R¹², R¹³ und R¹⁴, einer der Reste R^{9'}, R²³, R²⁴, R²⁵ und R²⁶, einer der Reste R^{10'}, R¹⁹, R²⁰, R²¹ und R²² und einer der Reste R²⁷, R²⁸, R²⁹, R³⁰ und R³¹ eine Einfachbindung darstellt;
R³², R³³, R³⁴, R³⁵ und R³⁶ unabhängig voneinander Wasserstoffatome, Niederalkyl-, Niederalkoxy-, Hydroxygruppen, Halogenatome oder Z darstellen und R³⁷ ein Wasserstoffatom oder eine Aminschutzgruppe ist,, wobei Z eine Einfachbindung oder ein zweiwertiger Rest der Formel: 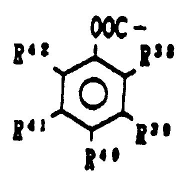 ist,
R³⁸, R³⁹, R⁴⁰, R⁴¹ und R⁴² unabhängig voneinander Einfachbindungen, Wasserstoffatome, Niederalkyl-, Niederalkoxy-, Hydroxygruppen oder Halogenatome sind,
mit der Maßgabe, daß immer einer der Reste R³², R³³, R³⁴, R³⁵ und R³⁶ Z ist und einer der Reste R³⁸, R³⁹, R⁴⁰, R⁴¹ und R⁴² eine Einfachbindung ist, wenn Z keine Einfachbindung ist, oder nur einer der Rest Z und R³⁸, R³⁹, R⁴⁰, R⁴¹ und R⁴² eine Einfachbindung ist, wenn zwei oder mehr der Reste R³², R³³, R³⁴, R³⁵ und R³⁶ Z darstellen;
oder Salze davon.

2. Arzneimittel zur oralen oder außeren Anwendung, das ein Thielocinderivat nach Anspruch 1 in Verbindung mit einem pharmazeutischen Träger umfaßt.

3. Arzneimittel nach Anspruch 2, umfassend eine Tagesdosis von 0,1 mg bis 500 mg eines Thielocinderivats nach Anspruch 1.

4. Arzneimittel nach Anspruch 3, das für die orale Anwendung geeignet ist und eine Tagesdosis von 0.5 mg bis 100 mg eines Thielocinderivats nach Anspruch 1 umfaßt.

5. Arzneimittel nach Anspruch 3 oder Anspruch 4 in einheitlicher Dosierungsform, umfassend eine bis fünf Portionen, die die Tagesdosis bilden.

6. Thielocinderivate nach Anspruch 1 zur Verwendung als Phospholipase-A₂-Inhibitoren.

## Revendications

1. Dérivés de la thiélocine ayant la formule: 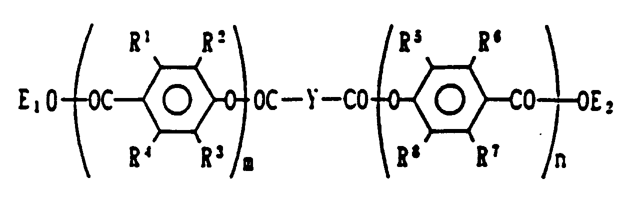 dans laquelle R¹, R², R³, R⁴, R⁵, R⁶, R⁷ et R⁸ sont indépendamment un hydrogène, un alkyle inférieur, un alcoxy inférieur, un hydroxy ou un halogène;
E₁ et E₂ sont indépendamment un hydrogène ou un reste ester;
m et n sont indépendamment un nombre entier de 0 à 4;
-Y- est un groupe divalent qui est choisi dans le groupe constitué des radicaux suivants:
-CH₂CH₂-, -CH=CH- , 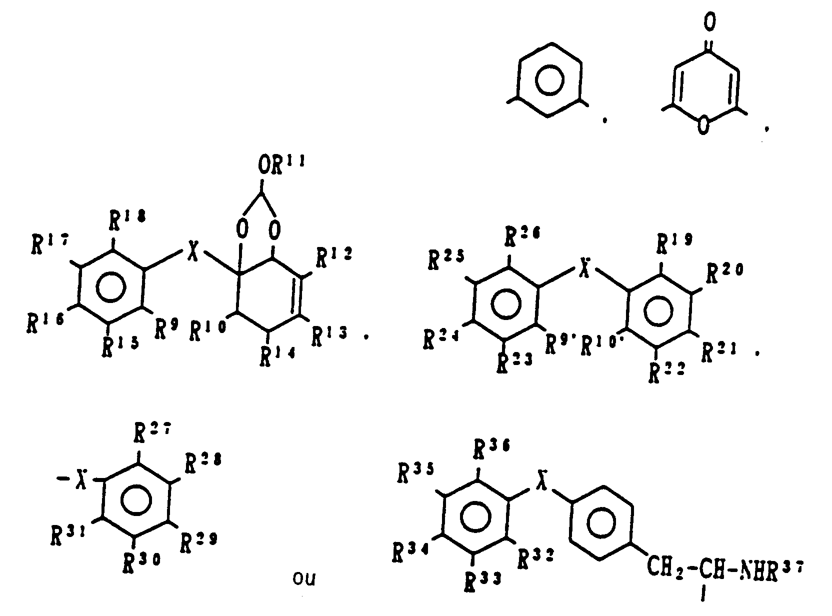 à condition que m + n ne soit pas égal à zéro lorsque Y est -CH₂CH₂-,
-CH=CH-, 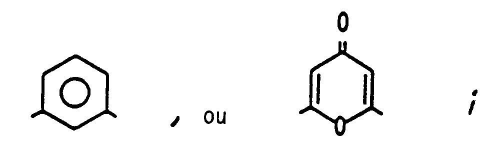 dans lesquels X est une liaison simple, CH₂, O, S, SO ou SO₂;
R⁹ et R¹⁰ sont indépendamment une liaison simple, un hydrogène, un alkyle inférieur, un alcoxy inférieur, un hydroxy ou un halogène, ou R⁹ et R¹⁰ peuvent être combinés ensemble pour former un méthylène, un éther, un sulfure, un sulfinyle ou une sulfone;
R^{9'} et R^{10'} sont indépendamment une liaison simple, un hydrogène, un alkyle inférieur, un alcoxy inférieur, un hydroxy ou un halogène, ou R^{9'} et R^{10'} peuvent être combinés ensemble pour former un méthylène, un éther, un sulfure, un sulfinyle ou une sulfone;
R¹¹ est un hydrogène ou un alkyle inférieur;
R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰ et R³¹ sont indépendamment une liaison simple, un hydrogène, un alkyle inférieur, un alcoxy inférieur, un hydroxy ou un halogène,
à condition que l'un de R⁹, R¹⁵, R¹⁶, R¹⁷ et R¹⁸, l'un de R¹⁰, R¹², R¹³ et R¹⁴, l'un de R^{9'}, R²³, R²⁴, R²⁵ et R²⁶, l'un de R^{10'}, R¹⁹, R²⁰, R²¹ et R²², et l'un de R²⁷, R²⁸, R²⁹, R³⁰ et R³¹ soit une liaison simple;
R³², R³³, R³⁴, R³⁵ et R³⁶ sont indépendamment un hydrogène, un alkyle inférieur, un alcoxy inférieur, un hydroxy ou un halogène, ou Z, et R³⁷ est un hydrogène ou groupe protecteur du groupe amino, où Z est une liaison simple ou un groupe divalent ayant la formule: 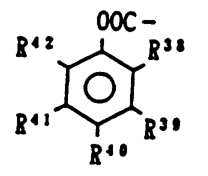 R³⁸, R³⁹, R⁴⁰, R⁴¹ et R⁴² sont indépendamment une liaison simple, un hydrogène, un alkyle inférieur, un alcoxy inférieur, un hydroxy ou un halogène,
à condition que l'un de R³², R³³, R³⁴, R³⁵ et R³⁶ soit toujours Z, et que lorsque Z n'est pas une liaison simple, l'un de R³⁸, R³⁹, R⁴⁰, R⁴¹ et R⁴² soit une liaison simple, ou que lorsque deux ou plus de R³², R³³, R³⁴, R³⁵ et R³⁶ dont Z, seulement l'un des Z et R³⁸, R³⁹, R⁴⁰, R⁴¹ et R⁴² soit une liaison simple;
ou les sels de ceux-ci.

2. Préparation pharmaceutique pour l'utilisation orale ou externe, ladite préparation comprenant un dérivé de la thiélocine selon la revendication 1 en association avec un support pharmaceutique.

3. Préparation pharmaceutique selon la revendication 2, comprenant une dose journalière de 0,1 mg à 500 mg de dérivé de la thiélocine selon la revendication 1.

4. Préparation pharmaceutique selon la revendication 3, appropriée pour d'administration orale et comprenant une dose journalière de 0,5 mg à 100 mg de dérivé de la thiélocine selon la revendication 1.

5. Préparation pharmaceutique selon la revendication 3 ou la revendication 4, sous forme de doses unitaires contenant de une à cinq parts constituant ladite dose journalière.

6. Dérivés de la thiélocine selon la revendication 1 pour utilisation comme inhibiteurs de la phospholipase A₂.
